# EUROPEAN PATENT SPECIFICATION

(11) **EP 0 950 062 B1**
(45) Date of publication and mention of the grant of the patent: **12.04.2006**
(21) Application number: 97927689.6
(22) Date of filing: 29.05.1997
(51) Int. Cl.: C07K 14/00, C07K 16/00, G01N 33/574, C07K 14/47, C07K 16/30, C12N 15/12

(54) **PROTEIN MARKERS FOR ESOPHAGEAL CANCER**
PROTEINMARKER FÜR ÖSOPHAGUSKREBS
MARQUEUR PROTEIQUE POUR CANCER DE L'OESOPHAGE

(30) Priority: 30.05.1996 US 18659 P
(43) Date of publication of application: 20.10.1999
(73) Proprietor: THE REGENTS OF THE UNIVERSITY OF MICHIGAN, Ann Arbor, Michigan 48109-1280 (US)
(72) Inventor: HANASH, Samir, M., Comprehensive Cancer Center, Ann Arbor, MI 48109-0752 (US); BEER, David, G., Comprehensive Cancer Center, Ann Arbor, MI 48109-0752 (US); KUICK, Rork, Comprehensive Cancer Center, Ann Arbor, MI 48109-0752 (US); HINDERER, Robert, Comprehensive Cancer Center, Ann Arbor, MI 48109-0752 (US)
(74) Representative: Lucas, Brian Ronald
(86) International application number: PCT/US1997/008656
(87) International publication number: WO 1997/045445

(56) References cited:
- VOLANT A. ET AL.: "p53 protein accumulation in oesophageal squamous cell carcinomas and precancerous lesions" JOURNAL OF CLINICAL PATHOLOGY, vol. 48, no. 6, June 1995 (1995-06), pages 531-534, XP000885863
- WU G. ET AL.: "Sucrase-Isomaltase gene expression in Barrett's esophagus and adenocarcinoma" GASTROENTEROLOGY, vol. 105, no. 3, September 1993 (1993-09), pages 837-844, XP000885867
- AL-KASSPOOLES M. ET AL.: "Amplification and over-expression of the EGFR and erbB-2 genes in human esophageal adenocarcinomas" INTERNATIONAL JOURNAL OF CANCER, vol. 54, no. 2, 8 May 1993 (1993-05-08), pages 213-219, XP000885871
- GARCIA-FONCILLAS J. ET AL.: "Overexpression of cyclin-D1 and cyclin-E is associated with poorer prognosis in nonmetastatic esophageal cancer" SUPPLEMENT TO GASTROENTEROLOGY , vol. 110, April 1996 (1996-04), page A516 XP000885845
- TANTRY L. ET AL.: "overexpression of receptors for Insulin-like growth factors (IGF) in human esophageal squamous carcinoma cells (ESC)" SUPPLEMENT TO GASTROENTEROLOGY, vol. 102, no. 4, April 1992 (1992-04), page A406 XP000885832
- HUANG T J ET AL: "Detection of 10 variants of biliverdin reductase in rat liver by two-dimensional gel electrophoresis." JOURNAL OF BIOLOGICAL CHEMISTRY, (1989 MAY 15) 264 (14) 7844-9. , XP000885831
- AMERICAN J. CLIN. PATHOL., 1992, Vol. 98, No. 2, SASANO et al., "The Proliferative Cell Fraction in Cytology Specimens", pages 161-166.
- MOL. IMMUNOL., 1983, Vol. 20, No. 12, PAK et al., "Identification and Isolation of a Common Tumor-Associated Molecule Using Monoclonal Antibody", pages 1369-1377.
- GASTROENTEROLOGY, 1992, Vol. 103, GRAY et al., "Epithelial Proliferation in Barrett's Esophagus by Proliferating Cell Nuclear Antigen Immunolocalization", pages 1769-1776.
- EXPERIMENTAL CELL RES., 1986, Vol. 162, VAN MUIJEN et al., "Cell Type Heterogeneity of Cytokeratin Expression in Complex Epithelia and Carcinomas as Demonstrated by Monoclonal Antibodies Specific for Cytokeratins Nos. 4 and 13", pages 97-113.
- CELL, December 1982, Vol. 31, Part 2, WU et al., "The Mesothelial Keratins: a New Family of Cytoskeletal Proteins Identified in Cultured Mesothelial Cells and Nonkeratinizing Epithelia", pages 693-703.
- ELECTROPHORESIS, 1994, Vol. 15, WIRTH et al., "Two-Dimensional Polyacrylamide Gel Electropheresis in Experimental Hepatocarcinogenesis Studies", pages 358-371.

## Description

### Background of the invention

### Field of the Invention

The present invention relates to proteins which are markers for esophageal cancer.

Esophageal cancer is a deadly disease, and one of the ten most common cancers worldwide. For reasons that are not clear, there has been a rapid increase in the incidence of esophageal adenocarcinoma. The occurrence of a change in the esophagus, referred to as Barrett's mucosa, in which a premalignant metaplastic epithelium replaces the normal squamous epithelium following gastroesophageal reflux, is the major risk factor for this deadly cancer. Reducing the high mortality associated with this cancer will require simplified means of diagnosing Barrett's mucosa and identifying, at an early stage, changes in Barrett's mucosa that indicate progression to cancer.

### Description of Related Art

At the present time, esophageal cancer is diagnosed primarily by biopsy. Unfortunately, by the time the cancer is diagnosed it is often far advanced. Survival after diagnosis is poor.

Thus, a need exists for the diagnosis of esophageal cancer at an early stage, or even a pre-malignant stage. Markers which correspond to the advance of the illness may be used to monitor therapeutic regimens.

### Summary of the Invention

By comparison of 2-D gels showing proteins from normal esophagus, normal gastric mucosa, Barrett's mucosa, and esophageal cancer, a set of proteins have been identified in the different source tissues. These proteins provide information on the pathogenesis of esophageal cancer, and may have utility as markers to monitor therapeutic regimens.

The proteins can also be purified and used as immunogens to generate antibodies which can be used as diagnostic reagents. Antibodies which specifically bind the proteins can be used in an immunoassay of body fluids to detect the proteins, thereby providing diagnostic information and/or information to monitor therapeutic regimens. The body fluids used in the immunoassay method may be samples of blood or serum, urine, saliva, tears, or other body fluids.

In addition, the proteins and products derived therefrom have therapeutic applications.

The invention is defined by the appended claims.

### Brief Description of the Drawings

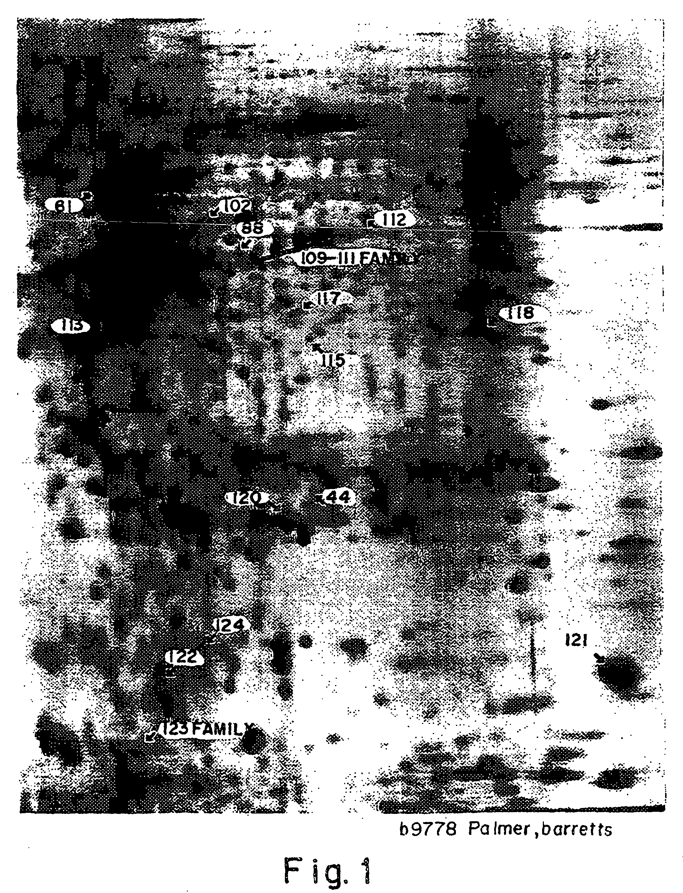

Figure 1 shows an entire carrier ampholyte (CA) gel with many important spots labeled.

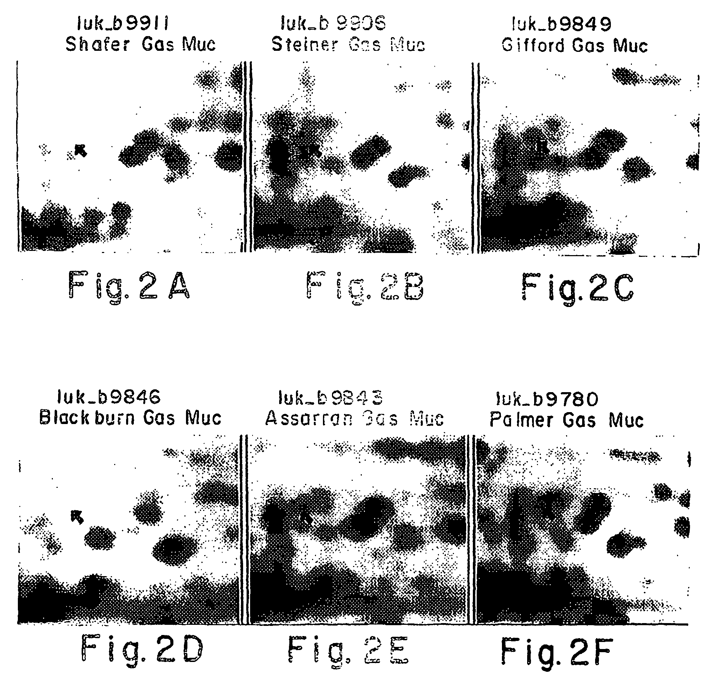

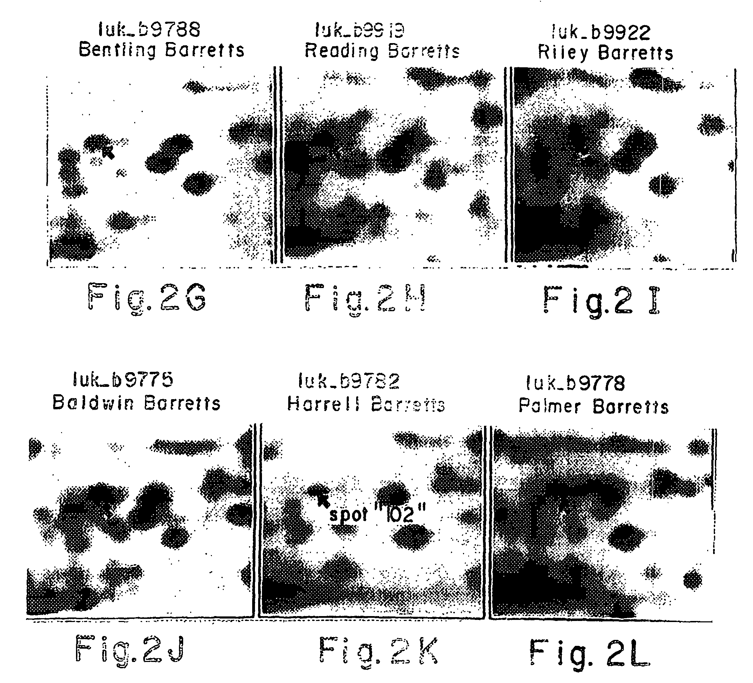

Figure 2 shows the region around spot 102 for CA gels from 12 samples.

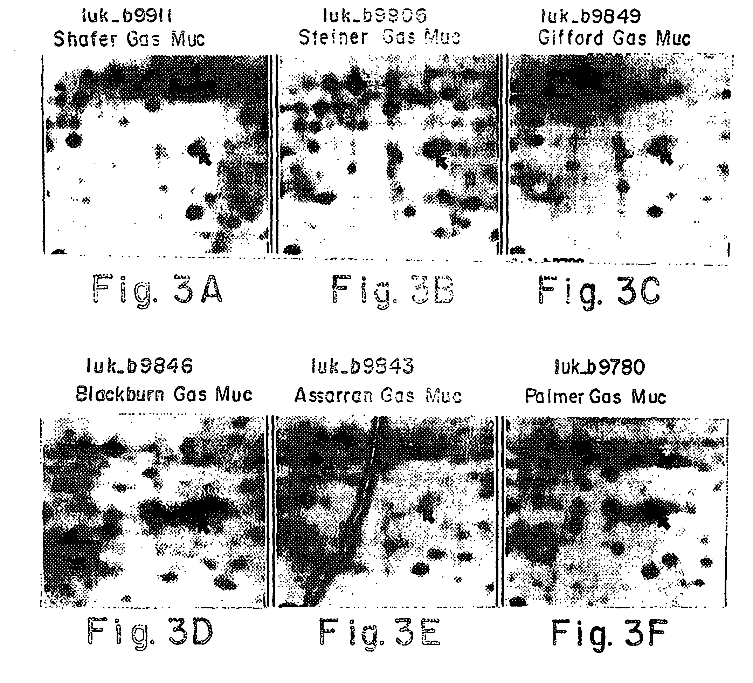

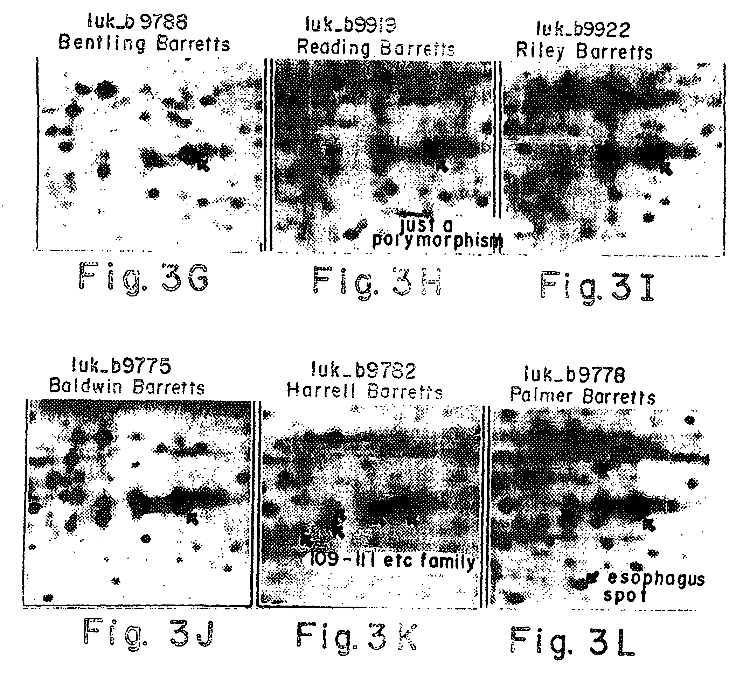

Figure 3 shows the region around spots 109-111 for CA gels from 12 samples.

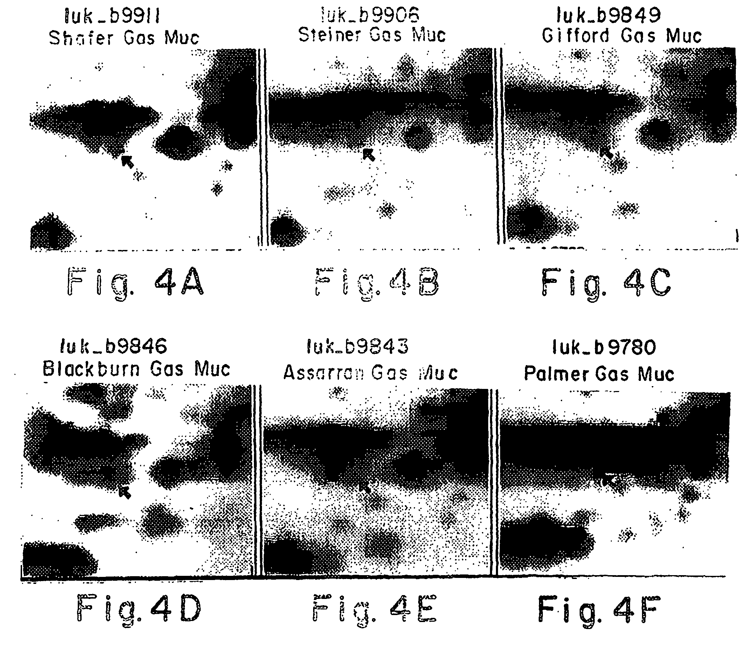

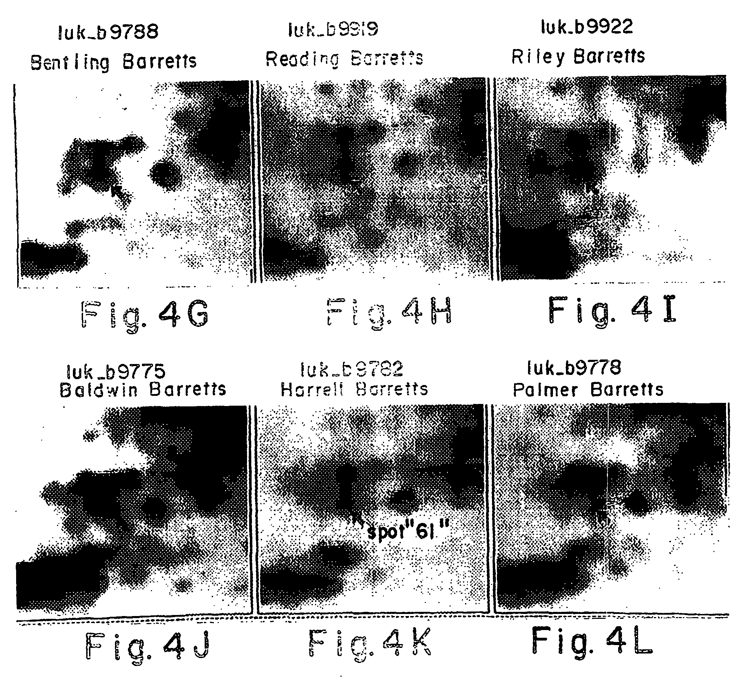

Figure 4 shows the region around spot 61 for CA gels from 12 samples.

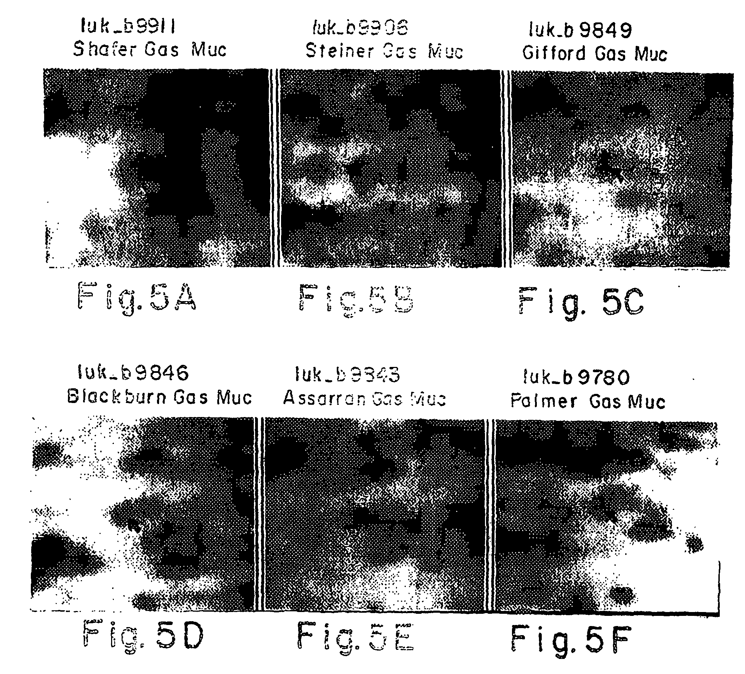

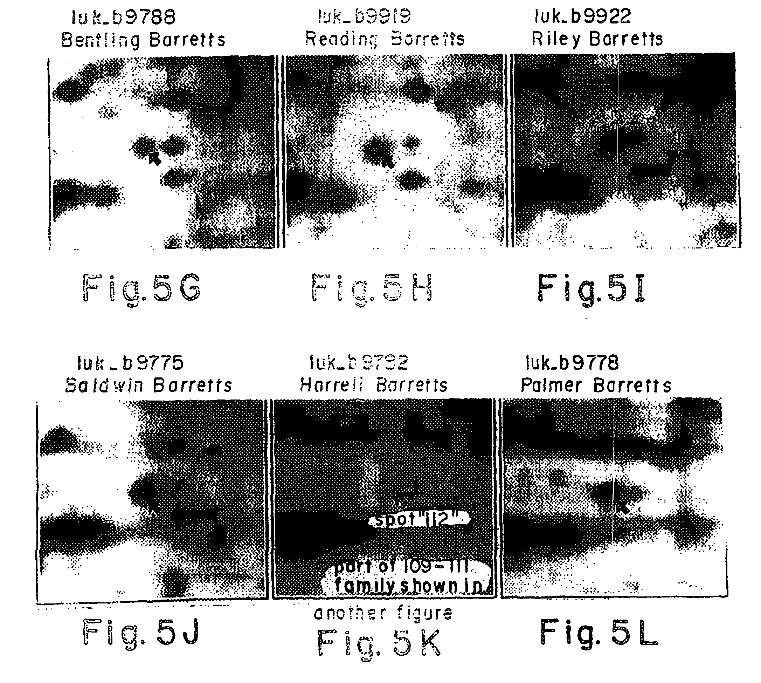

Figure 5 shows the region around spot 112 for CA gels from 12 samples.

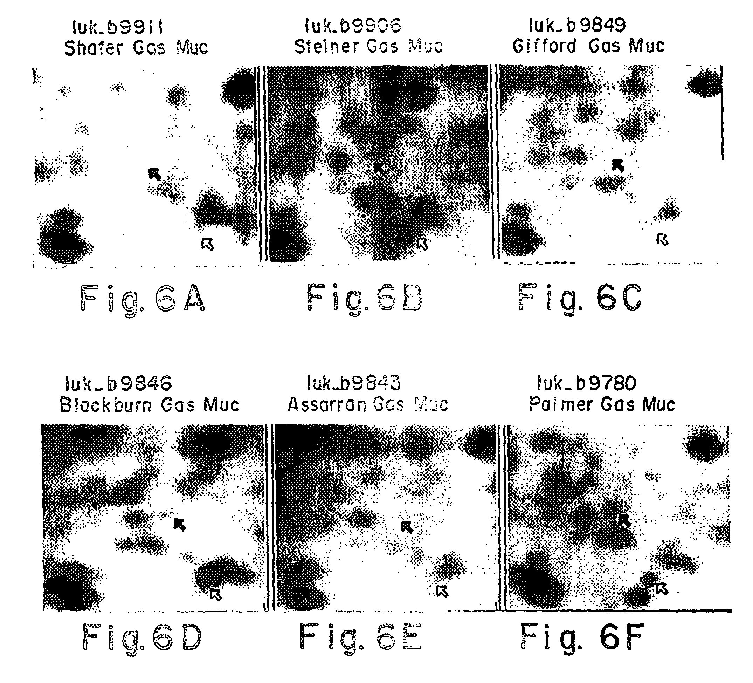

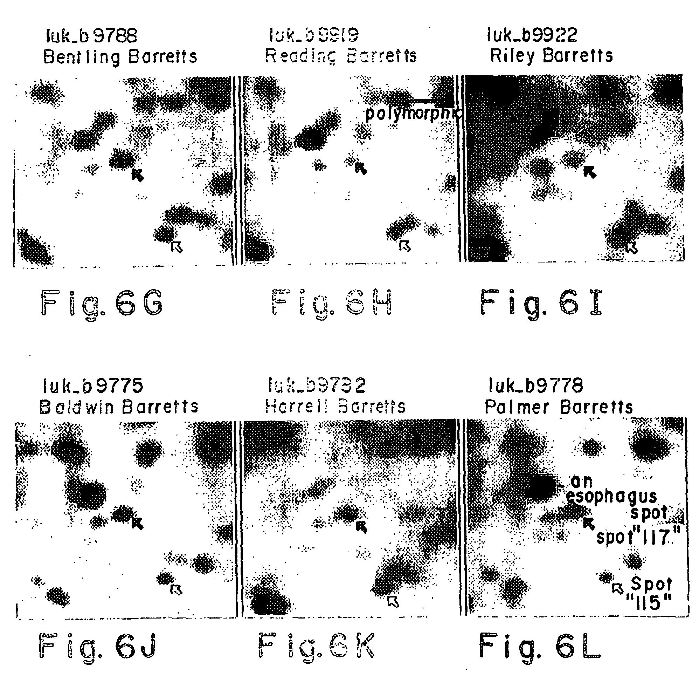

Figure 6 shows the region around spots 115 and 117 for CA gels from 12 samples.

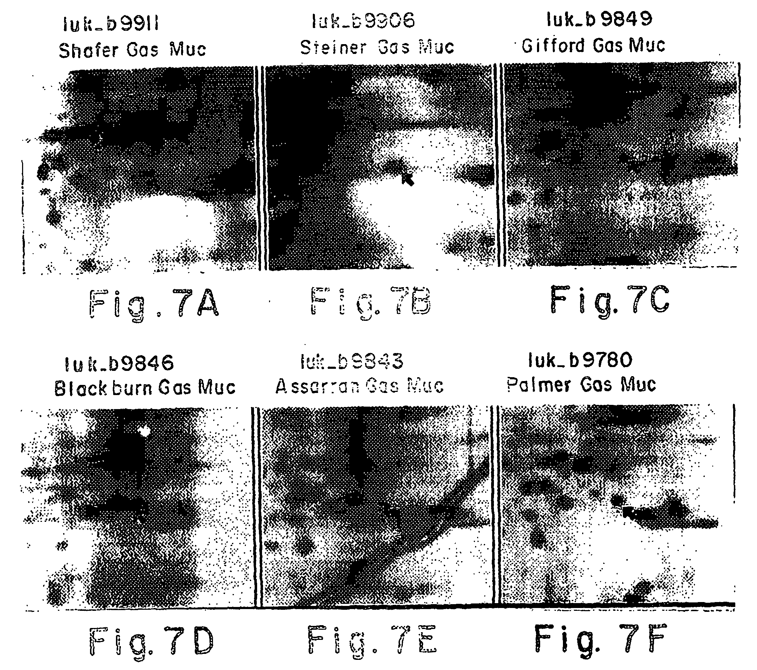

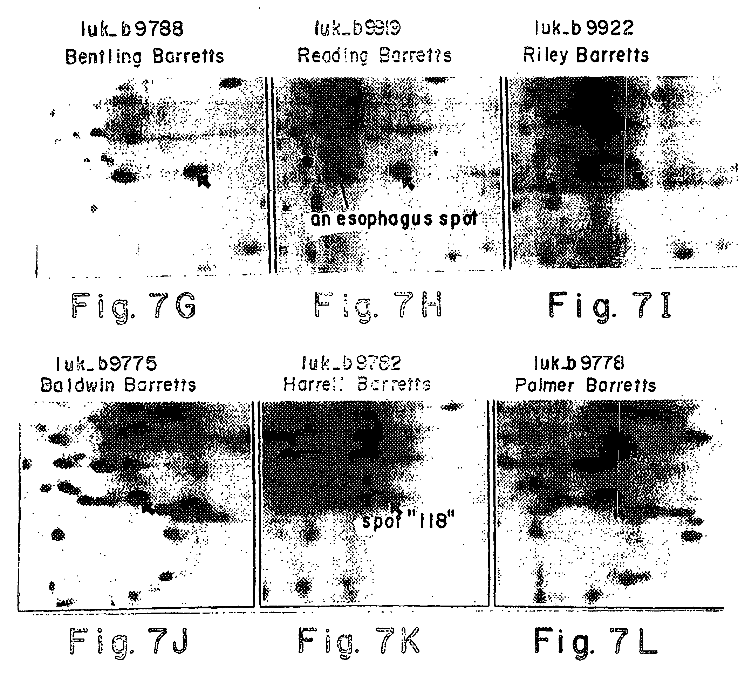

Figure 7 shows the region around spot 118 for CA gels from 12 samples.

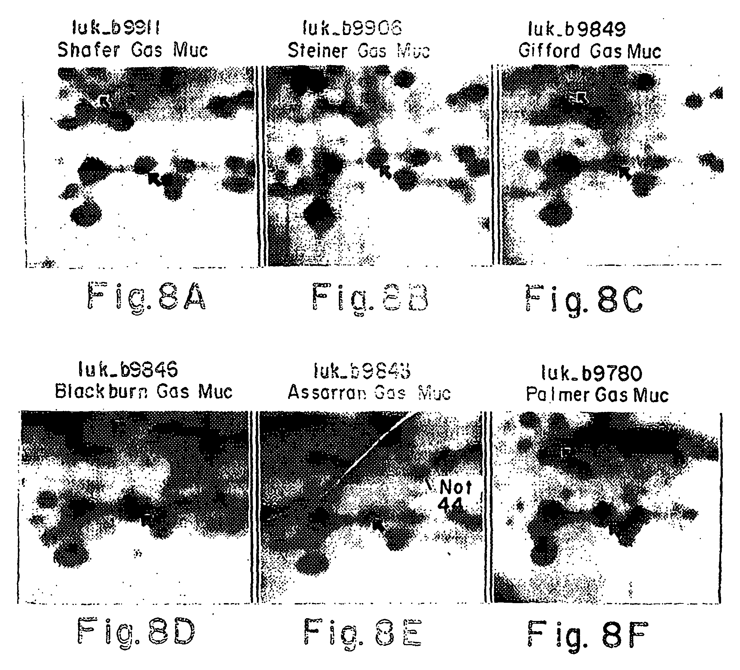

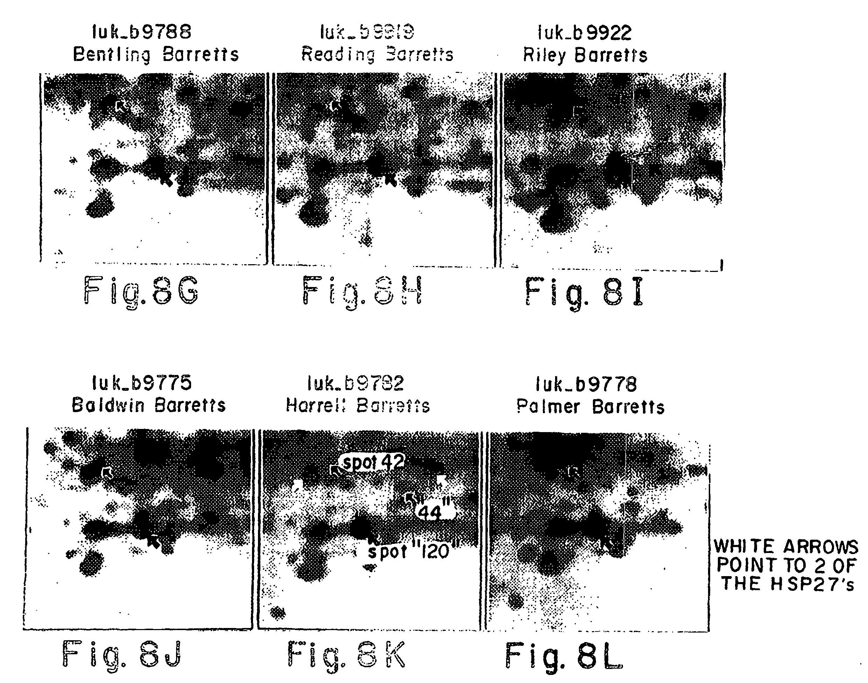

Figure 8 shows the region around spots 42, 44, and 120 for CA gels from 12 samples.

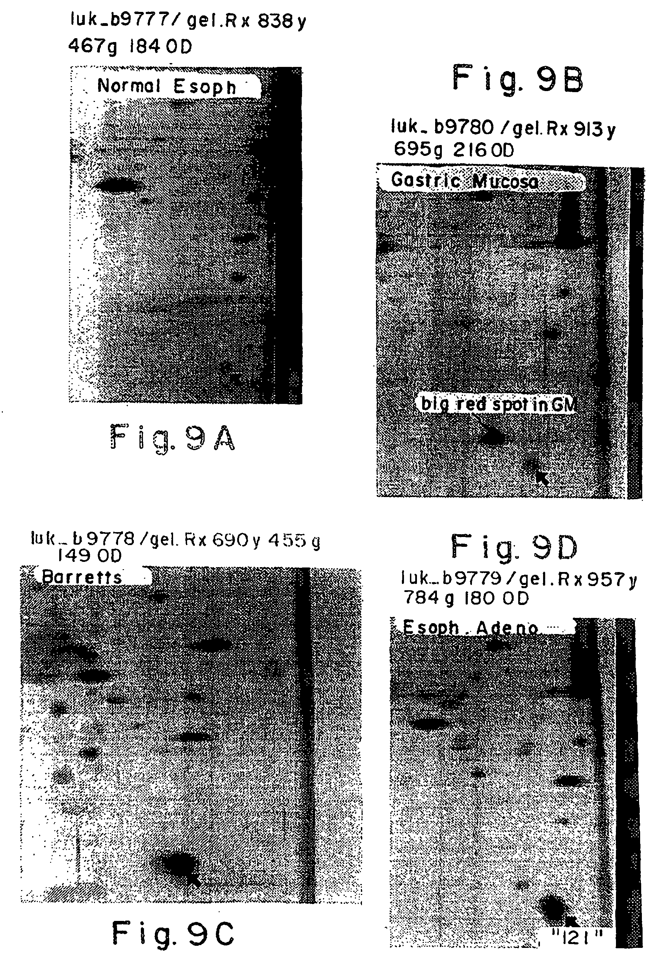

Figure 9 shows the region around spot 121 for CA gels from 4 samples.

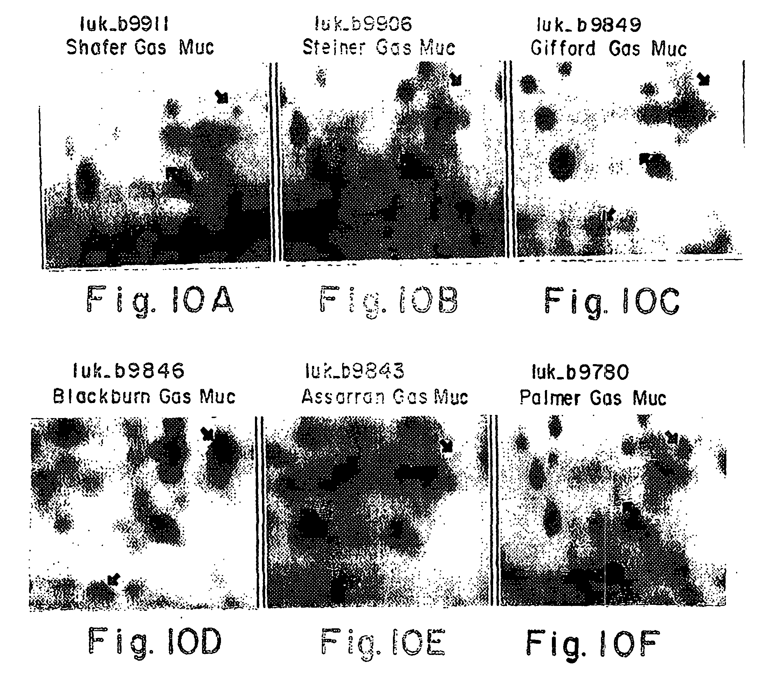

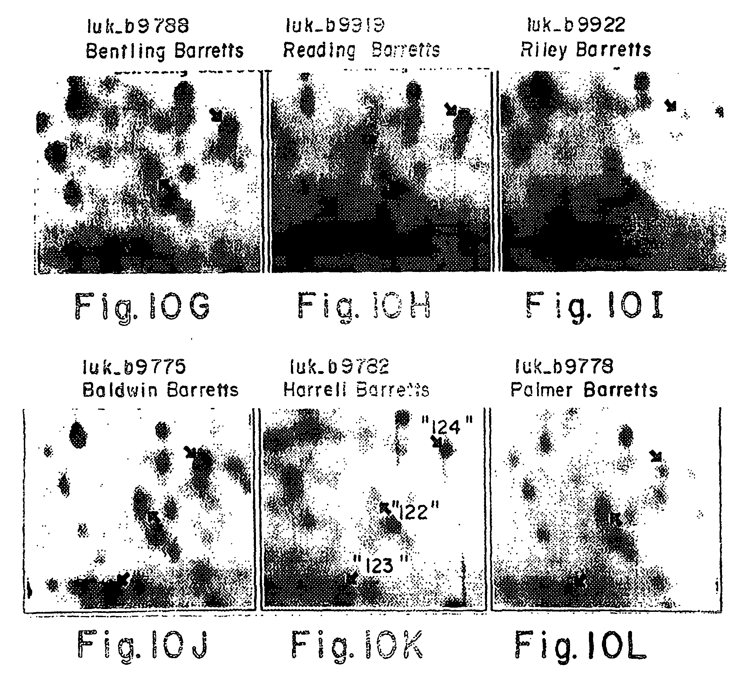

Figure 10 shows the region around spots 122, 123, and 124 for CA gels from 12 samples.

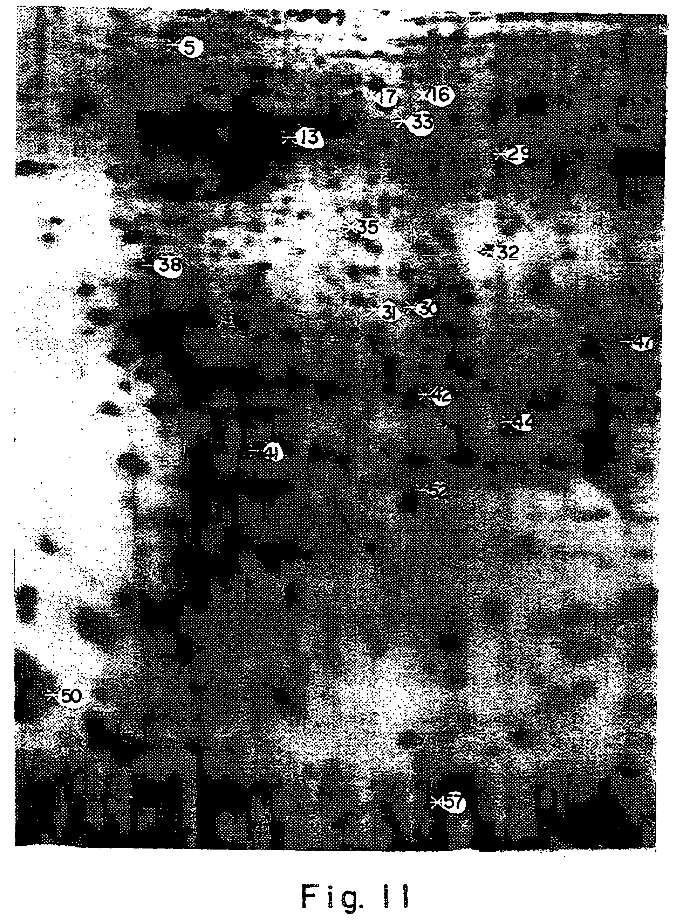

Figure 11 shows one quadrant of a gel.

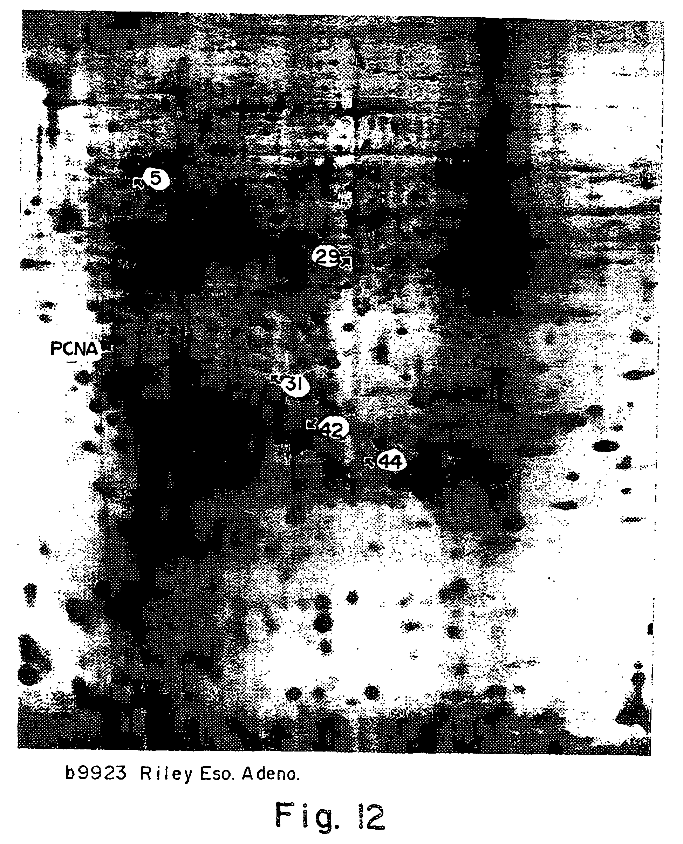

Figure 12 shows one quadrant of a gel.

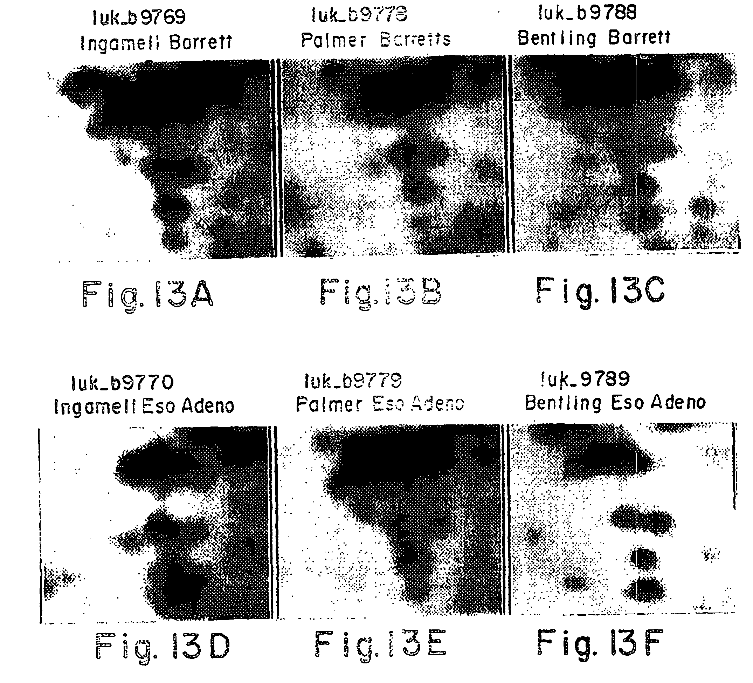

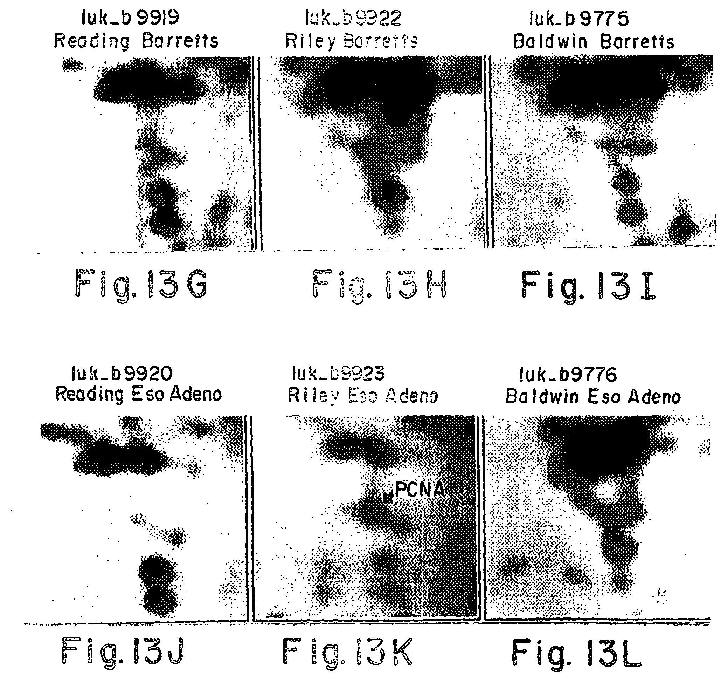

Figure 13 shows the region around spot PCNA for CA gels from 12 samples.

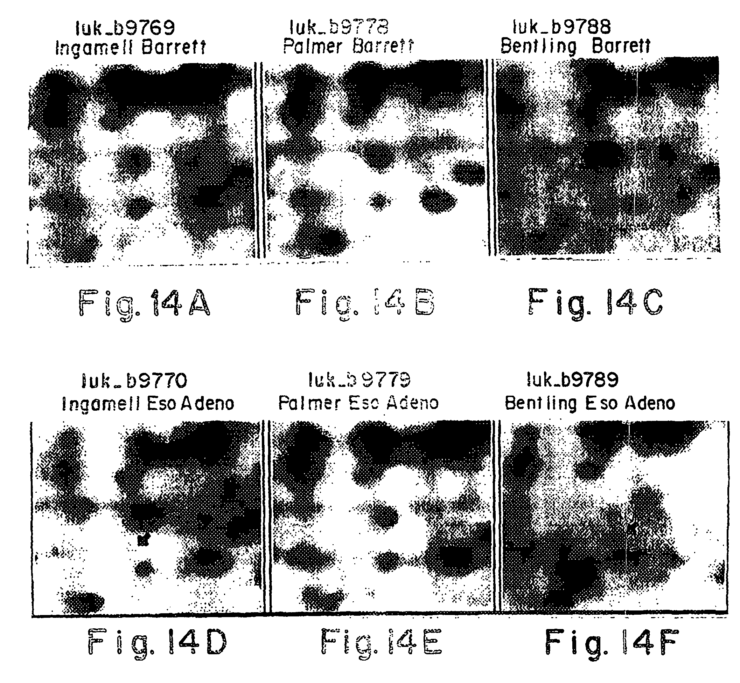

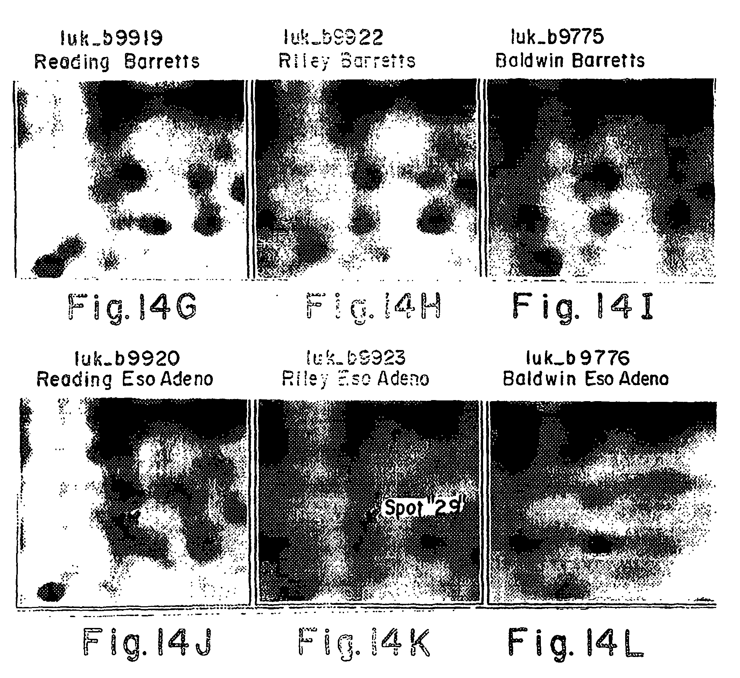

Figure 14 shows the region around spot 29 for CA gels from 12 samples.

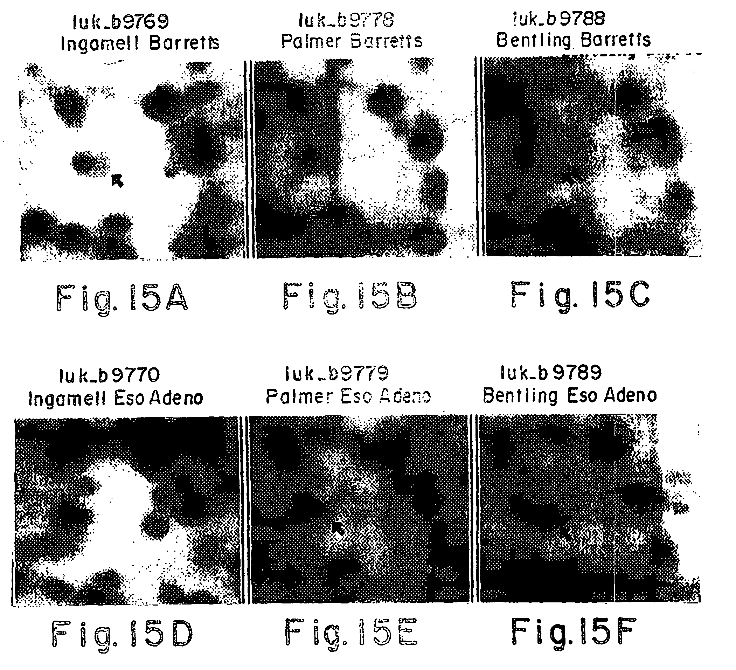

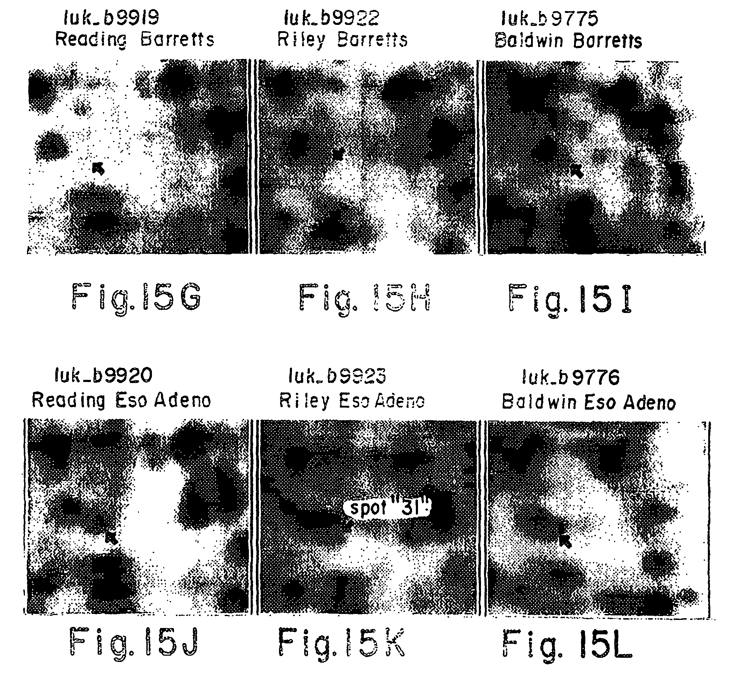

Figure 15 shows the region around spot 31 for CA gels from 12 samples.

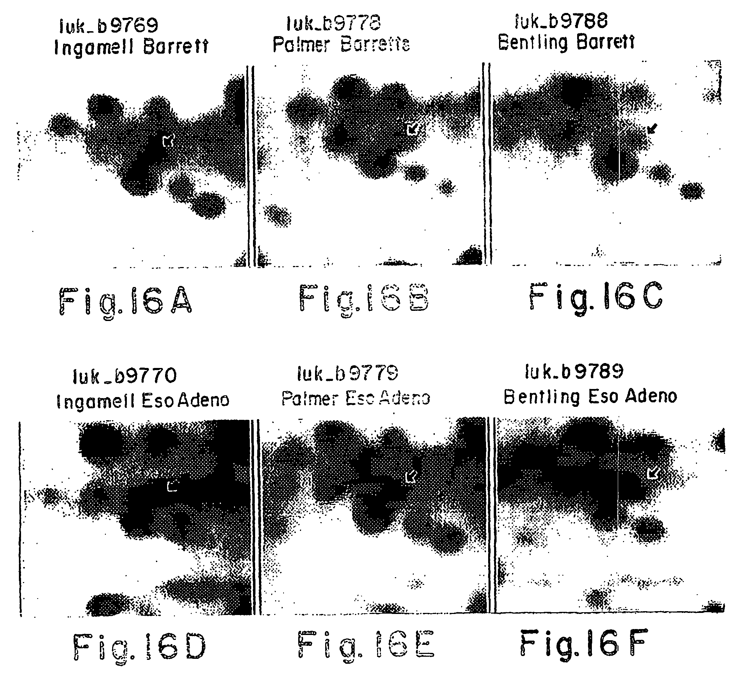

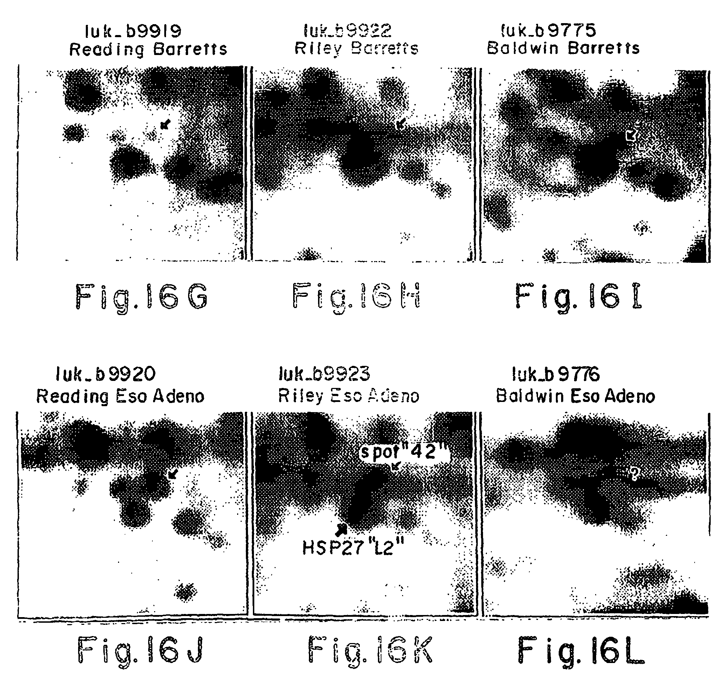

Figure 16 shows the region around spot 42 for CA gels from 12 samples.

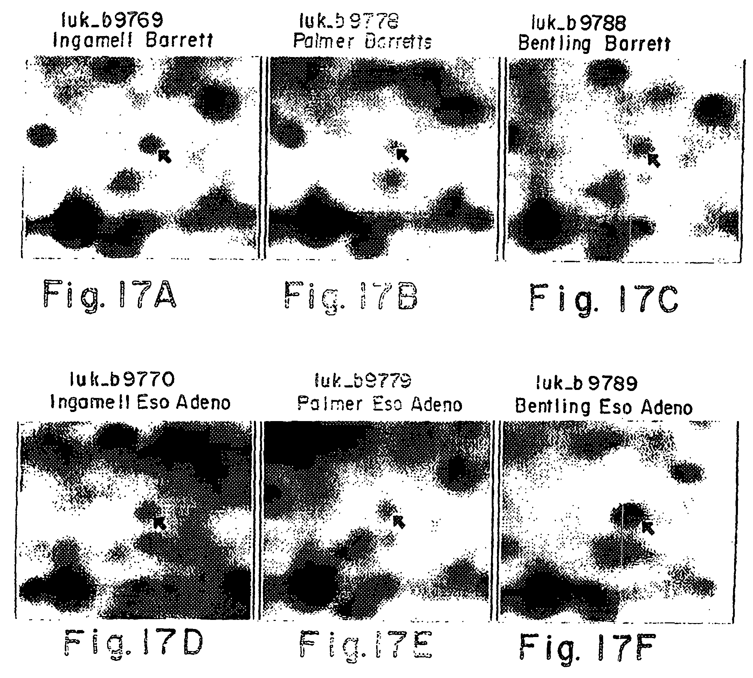

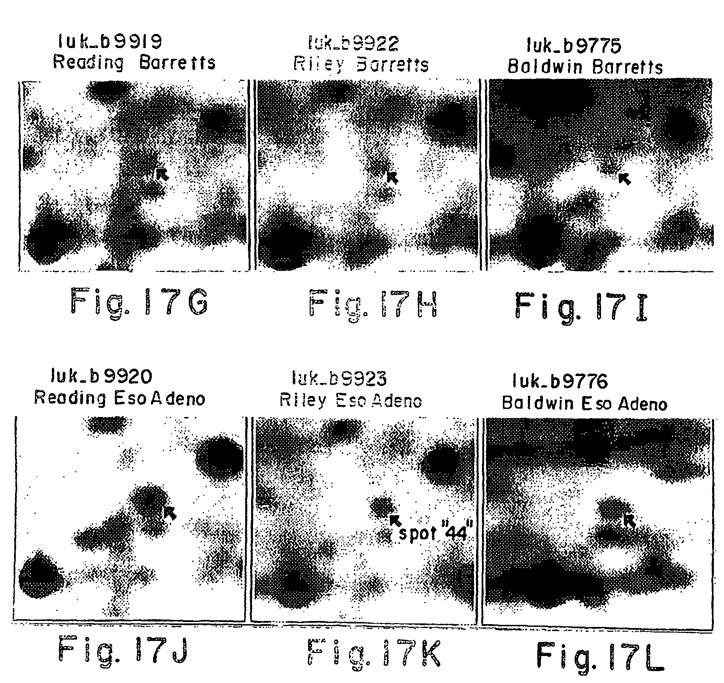

Figure 17 shows the region around spot 44 for CA gels from 12 samples.

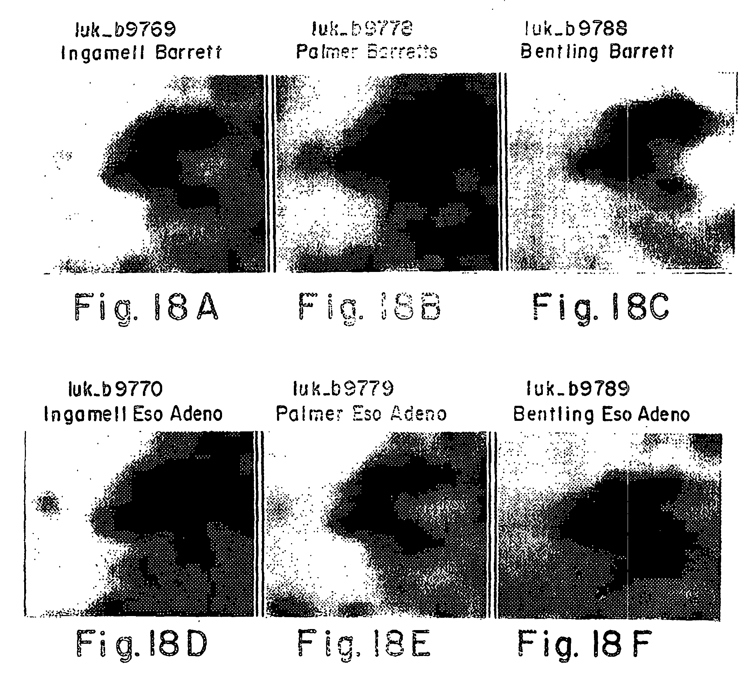

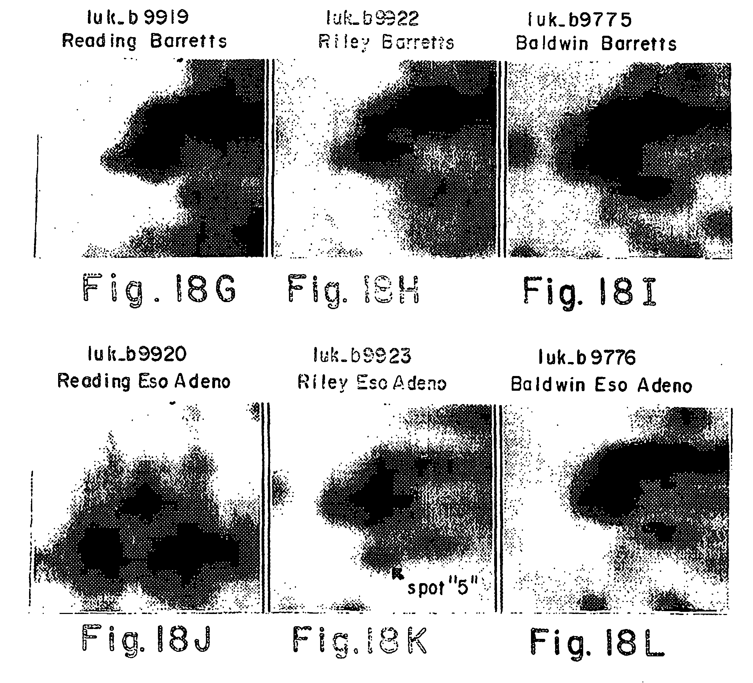

Figure 18 shows the region around spot 5 for CA gels from 12 samples.
Figure 19 shows one quadrant of an immobilized pH gradient (IPG) gel.
Figure 20 shows one quadrant of an IPG gel.

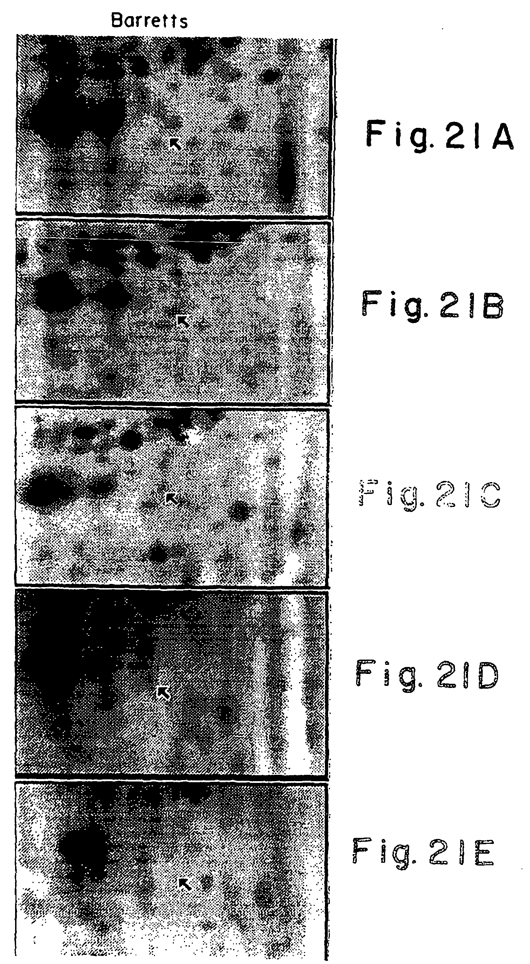

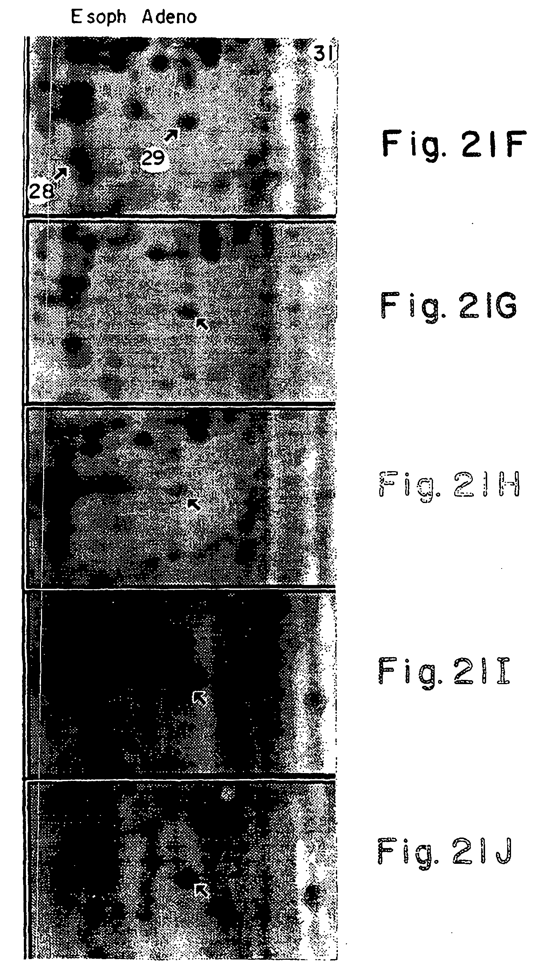

Figure 21 shows the region around spot 29 for IPG gels from 10 samples.

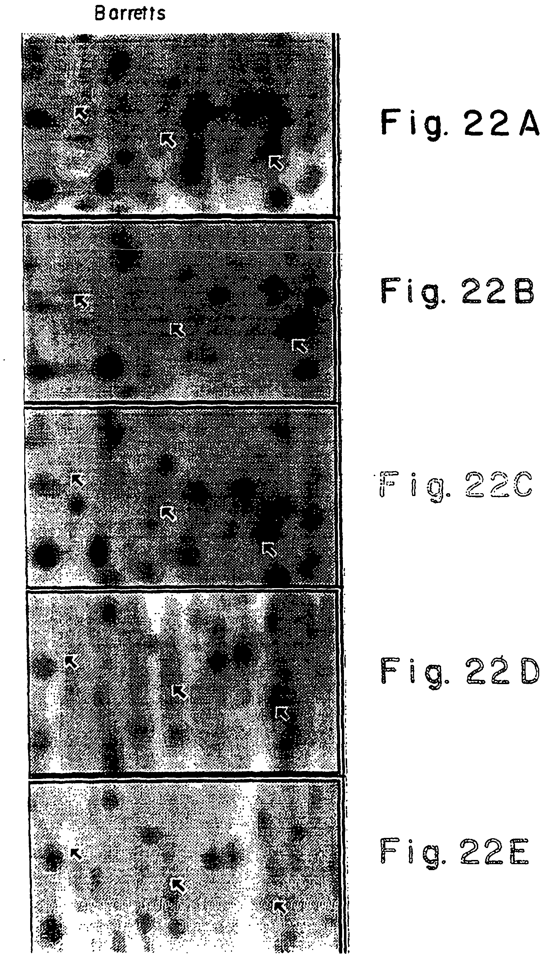

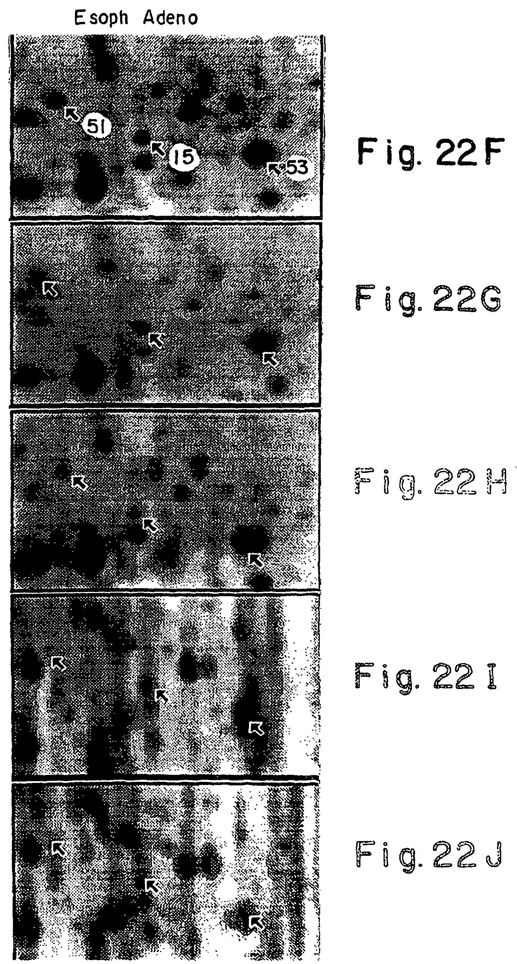

Figure 22 shows the region around spots 15, 51, and 53 for IPG gels from 10 samples.

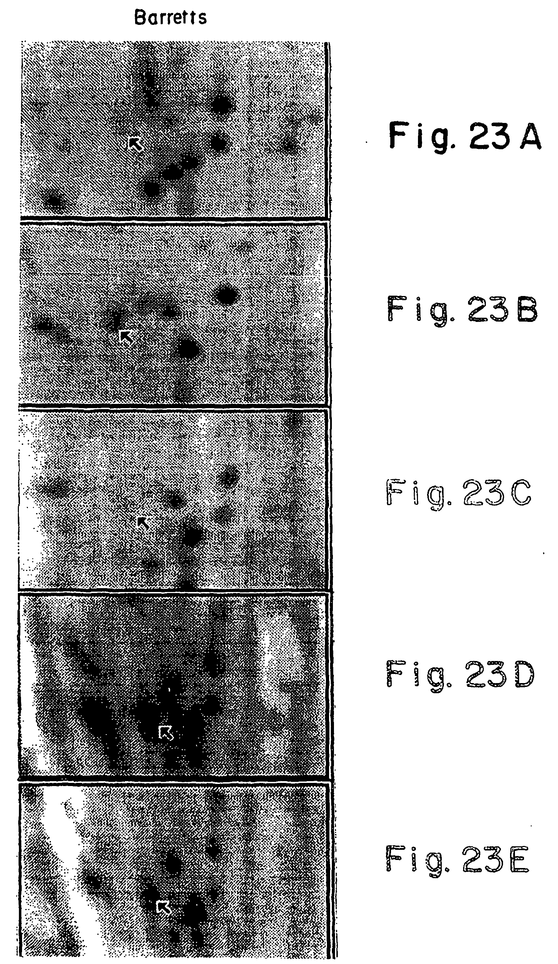

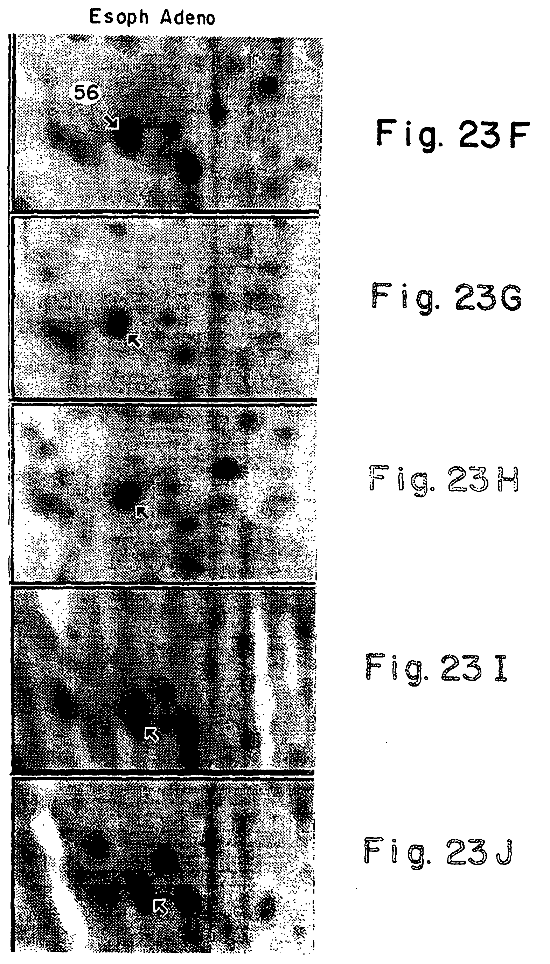

Figure 23 shows the region around spot 56 for IPG gels from 10 samples.

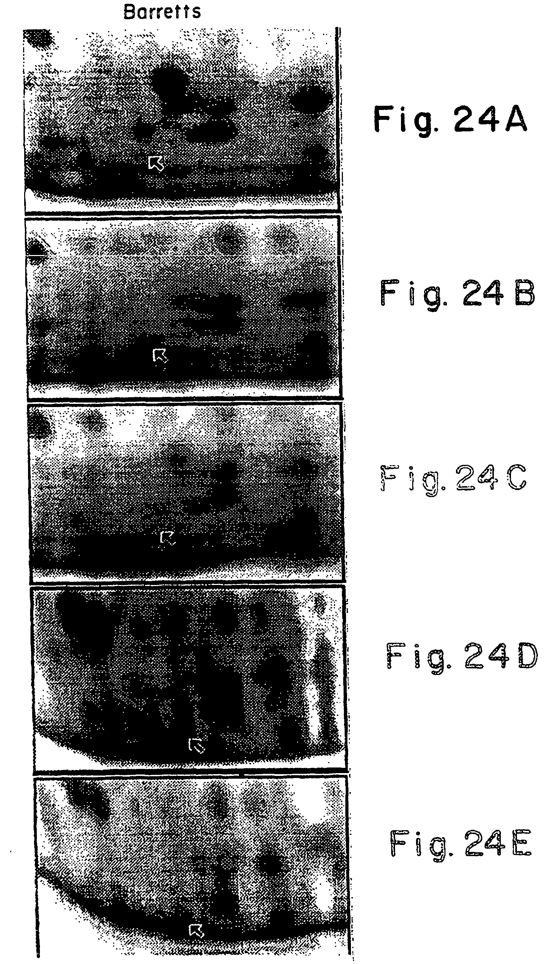

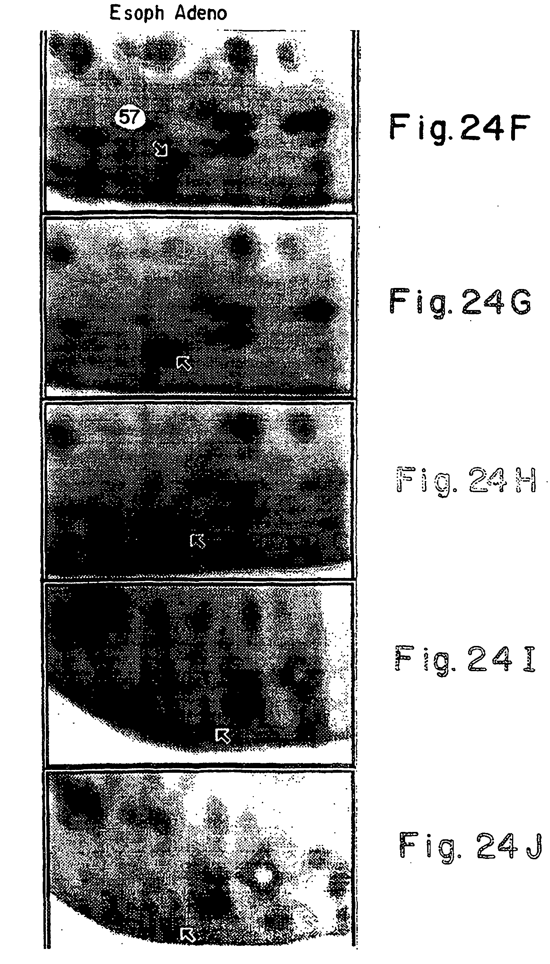

Figure 24 shows the region around spot 57 for IPG gels from 10 samples.

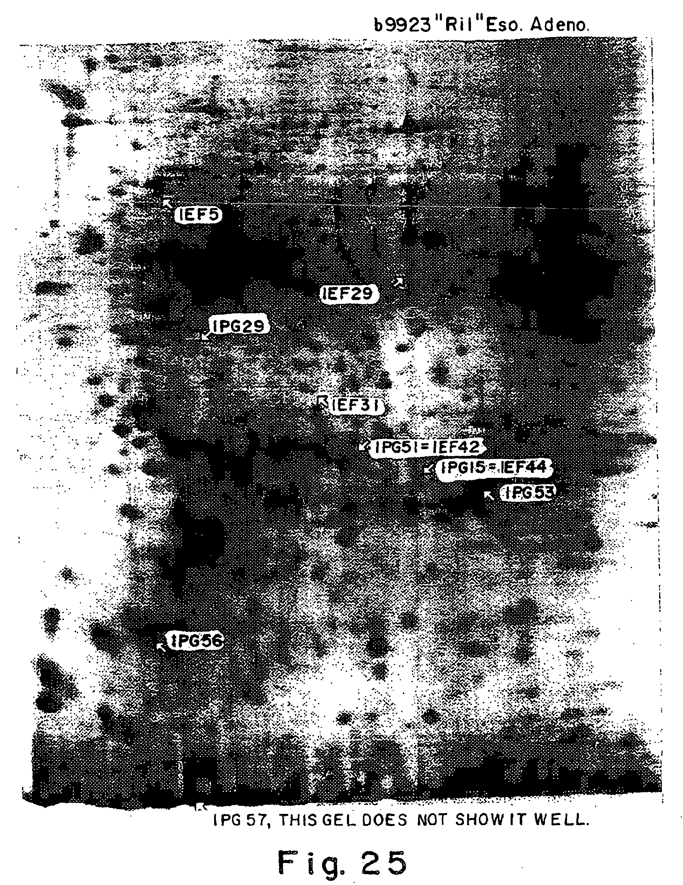

Figure 25 shows one quadrant of an IPG gel.

### Detailed Description of the Invention

### Example 1

### Carrier ampholyte (CA) based 2-D gels of esophageal cancer

Tissue from 27 patients was obtained for 2-D analysis from tumor tissue and normal esophagus. For patients in whom the tumor occurred in the context of Barrett's mucosa, Barrett's tissue was also obtained. (An interesting pathological feature of Barrett's mucosa is that it resembles gastrointestinal type of tissue rather than esophageal tissue, where it is found. The lower end of the esophagus resembles gastric mucosa rather than true esophageal tissue so that tumors that arise in that location need to be compared to normal esophagus as well as gastric tissue as control.) For tumors that occurred at the lower end of the esophagus, normal gastric mucosa was also obtained. All specimens were obtained at the time of initial surgery. Therefore, for every patient two control tissues were obtained, in addition to the cancer: normal esophagus and Barrett's for half of the patients, and normal esophagus and stomach for the other half.

Carrier ampholyte-based 2-D gels that cover the pH range of approximately 3.5-10.0 were prepared for all specimens.

Tissue was solubilized by addition of lysis buffer consisting of (per liter) 8 M urea, 20 ml of Nonidet P-40 surfactant, 20 ml of ampholytes (pH 3.5-10), 20 ml of 2-mercaptoethanol, and 0.2 mM of phenylmethylsulfonyl fluoride in distilled deionized water. Approximately 30 µl aliquots containing 70 µg of protein were loaded on individual gels.

Because isoelectric focusing is sensitive to charge modification, it is important to minimize protein alternations (e.g., proteolysis, deamidation of glutamine and asparagine, oxidation of cystine to cystic acid, carbamylation) that can result from improper sample preparation. Once solubilized, samples may be stored frozen at -80°C for short periods (<1 month) without significant protein modification).

2-D PAGE was done as previously described (Strahler et al, Joumal of Clinical Investigation, 85:200-207, 1990). In most cases aliquots were immediately applied onto isofocusing gels. First-dimension gels contained 50 ml of ampholytes per liter (pH 3.5-10). Isofocusing was done at 1,200 V for 16 h and 1,500 V for the last 2 h. 20 gels were run simultaneously. For the second-dimension separation, an acrylamide gradient of 11.4-14.0 g/dl was used. Protein spots in gels were visualized by the silver-staining technique of Merril et al. (Merril et al, Science, 211:1437-1438, 1981).

### Example 2

### Immobilized pH gradient (IPG) 2-D gels of esophageal cancer

In addition to generating 2-D patterns that were carrier ampholyte-based, a second set of patterns using immobilized pH gradients were generated as a complementary set.

Samples were prepared as for the CA gels discussed in Example 1. For first dimension separation an immobilized pH gradient covering the separation range of pH 4-10. The second dimension is the same as for the CA gels of Example 1.

IPG gels are prepared using derivatives of acrylamide having carboxyl or tertiary amino groups with specific pK values. A linear pH gradient is prepared from a dense, acidic solution and a light, basic solution using a two-chamber microgradient former. The pH gradient is stabilized during polymerization of the Immobiline-acryl-amide-bisacrylamide matrix by a colinear gradient of glycerol. Formulations of buffering Immobiline mixtures with titrating Immobiline for the pH limit solutions for narrow pH gradients (1 pH unit) or for broad pH gradients (>1 pH unit, up to 6 pH units) (Gianazza et al, Electrophoresis 6:113 (1985) and LKB application Note 324 (1984)) have been published.

The second dimension separates proteins on the basis of molecular weight in an SDS gel. An 11.5 to 14% T (2.6% cross-linking) acrylamide gradient provides effective separation of proteins of mass from 15,000 to 100,000. Proteins outside this range are less well resolved. Proteins with molecular weight less than 10,000 Da electrophorese close to the dye front and are not resolved.

### Example 3

### Computer assisted analysis of 2-D gels

Each gel was scanned in a 1024 X 1024 pixel format, where each pixel can have one of 256 possible values representing different degrees of intensity. Spot lists for study images are matched to spot lists of master images so that the result is a hierarchy of matched protein spots. The purpose of the matching is to link the same polypeptide spot through the hierarchy to allow assessment of its present, quantitative variation and specificity , as described in Strahler et al, 1990 (J. Clin. Invest. Vol.85, pp200-207). For comparison, between gels, of the amount of individual proteins, an adjustment process is utilized. The integrated intensity of detected polypeptides, measured in units of optical density per square millimeter, is adjusted relative to the intensity of reference polypeptides that are ubiquitously expressed. The adjustment is made to compensate for any variation between gels due to protein loading or staining.

### Example 4

### Proteins that are overexpressed in Barrett's dysplasia compared to normal tissue.

The number of spot differences between Barrett's and normal esophagus was enormous. It was very clear that Barrett's samples were much more similar to gastric mucosa samples, so spots that were larger in Barrett's compared to Gastric mucosa were identified.

Some of the spots present in Barrett's and not in gastric tissue were present in normal esophagus, and these are probably less interesting. They appear at the bottom beginning with spot 6. It should be noted that the spots that are bigger in Barrett's are also big in esophageal adenocarcinoma, since the two sample types represent essentially the same tissue but differ with respect to malignancy status.

Spot 102 is bigger in Barrett's than in normal tissues analyzed.

The large spots 109,110,111 are nearly always present in larger amounts in Barrett's than in either gastric mucosa or normal esophagus. They are also large in esophageal adenocarcinoma.

Spot 33 is always a pretty big spot in Barrett's, and occasionally is about the same in gastric mucosa, but often it is smaller. It would not deserve mention, except that it appears to be larger in esophageal adenocarcinoma.

Spot 61 is nearly always bigger in Barrett's. A spot just to the right is usually larger in gastric mucosa.

Spot 112 is larger in most Barrett's

Spot 113 is larger in Barrett's, but also present as an obvious spot in gastric mucosa. (the spot just to the right of 113 is large in gastric mucosa).

Spot 117 is potentially interesting. It is difficult to find in many gastric mucosas because it is in an area of many small spots.

Spot 115 looks pretty good and is near 117.

Spot 118 is usually bigger in Barrett's. It is quite basic and therefore difficult to compare in all gels.

Spot 44 looks good, but has already been found as an interesting spot because it seems bigger in esophageal adenocarcinoma.

Spot 120 is particularly large in Barrett's and esophageal adenocarcinoma.

Spot 121 is an enormous, gray, very basic, 18kD spot in Barrett's and esophageal adenocarcinoma. It is so far to the right on the gel that it is sometimes tough to figure out if it is there or off the gel.

Spot 122 is bigger in Barrett's.

Spot 123, very low and acidic, is part of a family of spots bigger in Barrett's.

Spot 124 looks good. It is possible that it is a phosphorylated P18 ("c").

Spots 6, 7, and 8 are bigger in Barrett's, but these are HUGE spots in normal esophageal.

Spot 116 is usually bigger in Barrett's but appears large in the esophagus.

HSP27 spots are bigger in Barrett's usually, but they are even larger in normal esophagus.

Spot 119 looked exciting, but it is a huge spot from the esophagus.

Summary of spots across Gastric Mucosa (GM), Cancer of the Cardia (CC), Barrett's (B), and Esophageal Adenocarcinoma (EA).

### Key:

- U=: interesting and up
- i=: somewhat interesting (up) but not as convincing as other spots.
- n=: not so interesting. For EA *vs* B it usually means the spots stay the same size (bigger than GM).
- D =: would mean down, but it is not needed for these spots.

The first 5 spots were first noticed comparing EA vs B, the remainder from comparing B vs GM. (Perhaps 115 can be noticed in EA vs B as well.)

| Spot# | B *vs* GM | EA *vs B* | CC *vs* GM | pH | MW |
|---|---|---|---|---|---|
| 5 | n | U | n | 4.4 | 57 |
| 29 | i | U | i | 6.1 | 42 |
| 31 | n | U | n | 5.7 | 33 |
| 42 | n | U | n | 6.2 | 25 |
| 44 | U | U | U | 5.9 | 27 |
| 33 | U | n | n | | |

the typical pattern: bigger in B, EA than GM, CC

| | | | | | |
|---|---|---|---|---|---|
| 61 | U | n | i | 4.4 | 58 |
| 113 | U | n | n | 4.4 | 41 |
| 102 | U | n | n | 5.7 | 51 |
| 109 | U | n | i | 6.0 | 48 |
| 110 | U | n | i | 6.1 | 49 |
| 111 | U | n | i | 6.2 | 50 |
| 112 | U | n | n | 6.3 | 53 |
| 117 | U | n | i | 6.1 | 41 |
| 115 | U | i | U | 6.1 | 39 |
| (even larger in EA than B, but not outstanding) | | | | | |
| 113 | U | n | n | 4.4 | 41 |
| 120 | U | n | n | 5.8 | 25 |
| 122 | U | n | n | 5.2 | 17 |
| 123 | U | n | i | 5.0 | 14 |
| 124 | U | n | n | 5.6 | 19 |
| 121 | U | | | 6.8 | 17 |

### Barrett's spots that can be confidently identified in IPGs:

The comparison of Barrett's to gastric mucosa and normal esophagus for the same patients described above identified some 15 spots that were thought to be increased in Barretts, using gels prepared with isoelectric focusing (IEF) in the first dimension. All of these except for one were present in equal or increased amounts in esophageal adenocarcinoma samples. Efforts have been made to identify these same protein spots in immobilized pH gradient (IPG) gels using as guides the spot location on IEFs, spot color using the same silver staining protocol, and whether a similar difference between Barrett's and control material (normal esophagus, gastric mucosa) was observed in IPGs.

Nine of the spots were confidently identified. They had spot numbers 113, 109-111 (a group of spots counted as a single protein), 117, 118, 120, 122, 123, 124, and 44 (which is among the spots reported as being larger in esophageal adenocarcinoma).

Spots 61 and 121 were also confidently identified. The remaining four protein spots (38, 102, 112, 115) were tentatively identified in IPGs.

### Changes observed in esophageal adenocarcinoma

This component of the study was initiated by analyzing the patterns of five patients that appeared to be the most outstanding. This is a comparison of Barrett's versus adenocarcinoma. The comparison led to the following findings:
- Spot 5: looks increased in EA,
- Spot 13: is increased in EA, I
- Spot 25: is up in most EA for which it there is good basic-side pattern.
- Spot 29: is up in EA in most patients, but is small and tough.
- Spot 30: is up in EA for most samples.
- Spot 31: is just left of 30, up in EA in all except one patient where it is down.
- Spot 32: is up in EA in most patients
- Spot 33: is up in EA in most patients
- Spot 35: is up in EA, but tough. Most clearly up in a couple of patients.
- Spot 38: is up in EA in every sample. It may be PCNA.
- Spot 40: is up in EA in two patients.
- Spot 41: is way up in EA in some patients. It also appears in esophagus though.
- Spot 42: is up in EA. One patient does not show it.
- Spot 44: is up in EA in most patients.
- Spot 47: It looks rather good.
- Spot 50: is up in EA, very acidic, and tough.
- Spot 52: is up in EA in all except one patient.

Subsequently other gels were reviewed for the above spot differences. All the gels were analyzed but the data was tabulated for only a subset of the clearest gels.

The table below shows only a subset of the spots above, in that it includes only the best spot differences. Asterisks appear next to those that were thought to be the most interesting among these.

### Symbols in the table mean:

+,++ = increases
-,- = decreases,
0 = no spot,
? = difficult area/tough to judge,
"s" = little if any difference in spot size.
Patients are identified by three letters.

| spot | Wil | Ray | Har | Sta | Sco | Bal | Ing | Pal | Ben | Rea | Ril |
|---|---|---|---|---|---|---|---|---|---|---|---|
| P18 | ++ | s | + | + | s | + | s | - | + | + | + |
| NDPK | ++ | s | + | s | s | s | + | - | + | + | + |
| 38= | | | | | | | | | | | |
| PCNA | ++ | s | s | s | + | s | ++ | + | + | + | + |
| 5* | + | + | s | + | s | s | + | + | + | ? | ++ |
| small but often nearly qualitative diff | | | | | | | | | | | |
| 13 | - | s | + | s | - | + | + | s | s | + | |
| near actin. not always up. | | | | | | | | | | | |
| 29* | 0 0 s | | | + | + | 0 | + | 0 | + | ++ | ++ |
| small but often near qualitative | | | | | | | | | | | |
| 31* | ++ | 0 | s | + | + | + | + | ++ | ++ | + | |
| A good spot never very large though. | | | | | | | | | | | |
| 35 | + | 0 | 0 | + | 0 | 0 | ? | ? | ? | + | + |
| might be good, but small and difficult. | | | | | | | | | | | |
| 42* | ++ | + | + | ++ | s | ? | + | ++ | ++ | ++ | |
| Good, sometimes big. Just up and right of "L2" hsp27 form. | | | | | | | | | | | |
| 44* | + | + | s | + | + | s/+ | - | s | + | + | + |
| Good, sometimes big always present in Barrett's though. | | | | | | | | | | | |
| 47 | + | ? | s | + | + | ? | - | + | + | + | ++ |
| Interesting. A rather streaky, rather basic spot. Hard, not that convincing. | | | | | | | | | | | |
| 50 | ? | 0 | + | + | s | + | s | s | + | + | + |
| A very low MW, very acidic protein. Fuzzy. Perhaps partly a function of better sample quality for the tumor samples. | | | | | | | | | | | |
| 57 | ++ | s | ? | + | + | - | - | + | + | + | + |
| A very low MW spot, sometimes large, but down in some. | | | | | | | | | | | |

### IPG gels comparing esophageal adenocarcinoma and Barrett's samples.

Initially acidic-loaded IPG's (immobilized pH gradient gels) were run for three patients (c0071-80) and basic-loaded IPG's for three different patients (clO25-34) and analyzed separately. As in previous experiments, there are many unique differences for each pair of samples from an individual, some due to varying amounts of various sample impurities, others perhaps idiosyncratic alterations which are true differences but are not shared among most tumors. The difficulty is therefore in finding differences shared among most tumors.

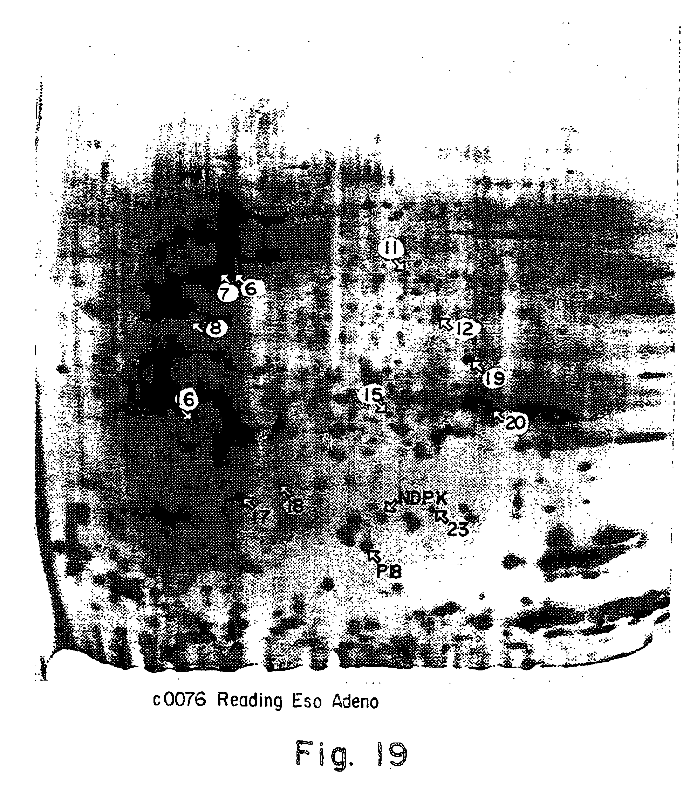

Figure 19 shows 12 spots that were larger in tumors compared to Barrett's in the acidic-loaded gels, for which the protein's identities are not known. (The known spots P18, and NDPKA also appeared larger in the tumors of these patients.). Some of these gels were rather light, so that the confidence in this set was not as great as for the basic-loaded gels, nor is the Est of candidate spots as large.

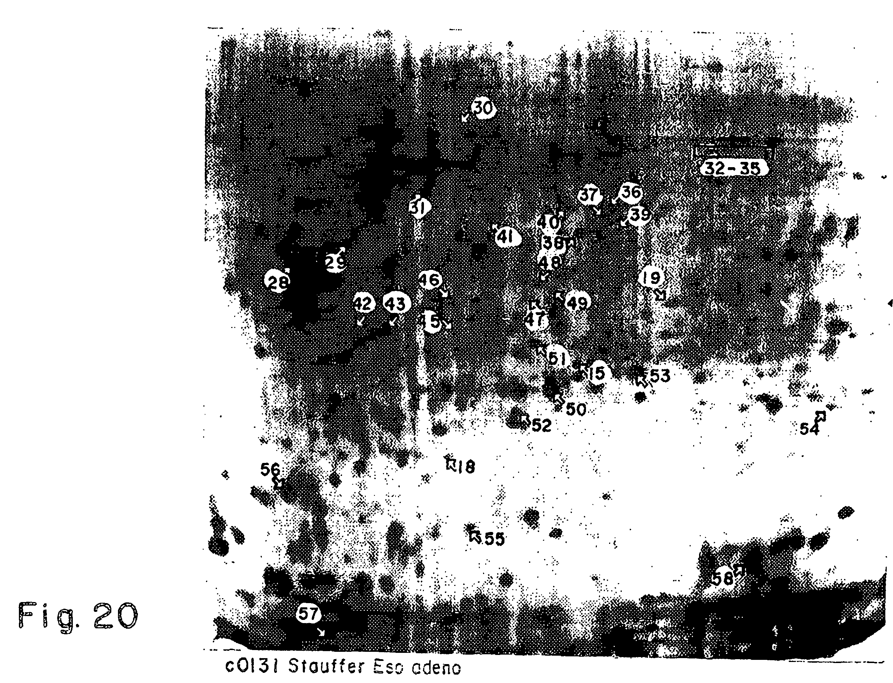

Figure 20 shows a much larger set of candidate spots thought to be larger in the basic-loaded IPG's. In fact there were so many differences that it caused some concern that a special subset of patients had been selected. The numbering system is consistent with the spot numbering in the acidic-loaded gels so that spots 15, 18, and 19 are the same spots as indicated in the first figure, however it is not always easy to relate spots between the two sets of gels loaded at opposite ends of the IPG strip.

Two new sets of basic-loaded IPG's were made from which the three best pairs (tumor, Barrett's) of patterns were selected in order to determine which candidate spots exhibited the most reproducible spot changes. From this review there appear to be six spots which are most worth pursuing for sequencing. That these spots are more worthy than some of the others is partially colored by their intensity in the tumors, as this relates to how easily they might be purified and sequenced. Also, in the case of spots 15 and 51 the fact that these spots were previously identified as interesting differences in IEF gels helped keep them on the list.

There are figures containing close-up sections of gels for three patients from one run, and two more from a second run (slightly different conditions), with Barrett's sample on the left and tumor sample on the right.
Spot 29 (approximately 35 kD) is reddish colored.
Spot 51 (28kD) has consistently been larger in tumor. On some runs the tumors have given large spots while sometimes it is not as big, suggesting that the protein may be difficult to solubilize.
Spot 15 (26kD) has been a very consistent difference, but may be challenging to sequence because the spot is often not very large.
Spot 53 (24kD) has some overlap with another spot which may or may not be related.
Spot 56 (2OkD) actual appears as a pair of spots which do seem related, as their sizes appear to be correlated.
Spot 57 (IOkD?) is a reddish, very low molecular weight spot. It does not show up very well in the bottom 2 pairs of gels in the figure because it is "in the dye front" a bit, but the difference is apparent on the actual gels.

### Most interesting spots in Esophageal Adenocarcinoma compared to Barrett's

It follows that studies were performed using two separate methods of first dimension separation, namely isoelectric focusing gels (IEFs) and Immobilized pH gradient gels (IPGs). We had first singled out 5 spots (IEF5, IEF15, IEF29, IEF31, IEF51) as the most consistent differences in IEF samples, where we searched for spots that were absent or very nearly absent in tissues other than the esophageal adenocarcinoma (Barrett's, gastric mucosa, normal esophagus). Subsequently in studies of IPGs, 6 very interesting spots were identified which were larger in the esophageal adenocarcinoma samples (IPG29, IPG56, IPG57, IPG53, IPG51, IPG15, the last two being identical to spots IEF42 and IEF44 respectively). Spots IEF5, IEF29, and IEF31 were tentatively identified in the IPG gels.

The table below summarizes which spots were increased (in both types of gels) in the esophageal adenocarcinoma compared to Barrett's. "+" indicates clear increase, "-" indicates no difference, and "?" implies difficulty scoring the spot, usually due to being obscured by another large spot, or in the case of spot IPG57 because the spot was not well separated from the dyefront (bottom) of the second dimension gel.

| | | | | | | | | | |
|---|---|---|---|---|---|---|---|---|---|
| patient gel | lPG29 type | IPG56 | lPG57 | IPG53 | IPG51 | IPG15 IEF5 =IEF42=IEF44 | | IEF29 | IEF31 |
| 1 Bal IPG | + | + | + | + | + | - | | | |
| IEF | + | + | + | + | - | + | - | - | + |
| 2 Ben IPG | + | + | + | - | - | + | | | |
| IEF | + | + | + | + | + | + | + | + | + |
| 3 Sta IPG | + | + | + | + | + | + | | | |
| IEF | + | + | + | + | + | + | + | + | + |
| 4 Rea IPG | + | + | + | + | + | . | | | |
| IEF | + | + | + | + | + | + | ? | + | + |
| 5 Sco IPG | + | + | ? | - | + | - | | | |
| IEF | - | + | + | - | - | + | - | + | + |
| 6 Wil IPG | + | + | + | + | + | + | | | |
| IEF | + | + | + | + | + | + | + | - | + |
| 7 Pal IPG | - | + | + | - - | | - | | | |
| IEF | - + + + + | | | | | - | + | - | + |
| 8 Har IPG | + | + | ? | - | + | + | | | |
| IEF | + | + | + | - | + | - | - | - | - |
| 9 Bit IPG | + | + | + | + | - | + | | | |
| IEF | + | + | + | + | - | + | ? | ? | ? |
| 10 Lab IPG | + | - | + | + | + | - | | | |
| IEF | + | - | + | + | + | - | ? | ? | ? |
| 11 Duc IPG | + | + | ? | - | + | + | | | |
| IEF | ? | + | + | + | + | + | + | + | + |
| 12 Ray | | | | | | | | | |
| IEF | - | - | ? | - | + | + | + | - | - |
| 13 Ril | | | | | | | | | |
| IEF | + | + | ? | + | + | + | + | + | + |
| Molecular | | | | | | | | | |
| Weight kD | 37 | 19 | 10 | 26 | 25 | 27 | 57 | 42 | 33 |
| pH | 4.7 | 4.3 | 4.6 | 6.4 | 6.2 | 5.9 | 4.4 | 6.1 | 5.7 |

Spot 56 actually appears as a pair of spots which do seem related, as their sizes appear to be correlated.

### Example 5

### Analysis of Secreted Proteins

Gels of secreted proteins were prepared from fresh tissue pieces from two new patients for normal esophagus, gastric mucosa, and Barrett's, as well as control gels separating proteins in the media used to culture the cells. These patterns of "secreted proteins" mixed with media proteins (serum) are promising. However, the limitation here is that this study requires fresh samples and therefore the number of samples is bound to be small. The results to date are that a group of spots numbered 109-111, which were found to be increased in Barrett's relative to normal esophagus and gastric mucosa, are quite large in the Barrett's"secreted proteins" samples compared to control samples.

### Example 6

### Sequencing

There are a total of at least some twenty spots that are of interest to sequence and identify. The spots are eluted from the gels and subjected to sequence analysis.

A number of potential markers described above on the basis of their estimated isoelectric point (pl) and molecular weight, have been further characterized with respect to their N-terminal sequence. This information has led to the identification of some of the proteins based on their matching with protein databases and to novel sequences for others. The data is shown below.

### Spots which have been sequenced

101, MW 52 kD, pH 5.8, cytokeratin 8, cleaved based on the sequence T?GPRAF??R
111, MW 50 kD, pH 6.2, albumin, cleaved
109-111 are a train of related spots. 11 identified as a cleaved albumin based on seqeunce
120, MW 25 kD, pH 5.8, albumin, cleaved also appears to be a cleaved serum albumin based on the seqeunce DAHKSEV
124, MW 19 kD, pH 5.6, possibly related to collagen sequence XXAPLTATAP. No match in data base but APLTAT matches part of collagen
29(IPG), MW 37 kD, pH 4.7, HS72 or HS7C human, cleaved seqeunce of GSTRIPKIQKLLQD, which is cleaved HS72 or HS7C human
42, MW 25 kD, pH 6.2, actin capping protein, cleaved gave seqeunce QVKGKKNIRATE, which is cleaved actin capping protein 53(IPG), MW 26 kD, pH 6.4, triose phosphate isomerase, cleaved appears to be triose phosphate isomerase based on seqeunce GNWKMNQR (and a minor sequence VGGNWKMN)
56(IPG), MW 19 kD, pH 4.3, no match for seqeunce gave sequence (ASQ)VFV(FG)RM?MK but nothing matches in the data base and may be novel
14, MW 50 kD, pH 5.8, moesin or ezrin internal seqeunce of moesin or ezrin based on seqeunce of (LHD)-MGNHELYM
139, MW 20 kD, pH 4.4, amyloid beta (A4) homolog 2 precursor may be cleaved amyloid beta (A4) homolog 2 precursor based on the seqeunce MYPELQV(?)D
157, MW 52 kD, pH 4.2, no match for seqeunce gave seqeunce K(?)HKGE(?)N(?)LLRAQP(small)EL. There are no data bases matches for this seqeunce
164-166, MW 46 kD, pH 4.4, vimetin, cleaved a train of related spots. Appears to be cleaved vimetin based on the seqeunce SSVPGVRLLQ
92, MW 5 kD, pH 5.1, possibly cytokeratin 17 based on amino acid analysis this was thought to be cytokeratin 17. Seqeunce analysis gave a weak signal of XTXILKFTL and ck17 has N-terminal seqeunce TTSIRQFTSS, some of which lines up
143, MW 56 kD, pH 5.6, possibly glutathione synthetase possible glutathione synthetase but the data from amino acid analysis is not strong enough to be certain.

### Example 7

### Antibody production

The proteins eluted from the gels as in Example 6, or peptide fragments thereof, may be used as immunogens for the production of antibodies. The antibodies may be polyclonal antibodies or may be monoclonal antibodies. The antibodies are made by methods known to those skilled in the art. Antigen-binding fragments of the antibodies are prepared by methods known to those skilled in the art. Antibodies or antibody fragments with very high affinity and specificity may be used for immunological tests for markers of esophageal cancer.

Antibodies or antibody fragments with very high affinity and specificity are used to test body fluids for the presence of the proteins identified above. The body fluids tested may be blood or serum, urine, saliva, tears or other body fluids. The immunoassay may be of any type known to those skilled in the art. The assay may be homogeneous or heterogeneous. The assay may be used with automated machines, or may be functional in lower technology environments.

Such a test provides a simple and rapid test to aid in diagnosis of these serious diseases.

### Example 8

### In vivo applications

The antibodies or antibody fragments produced in Example 7 may be conjugated with a radioactive tag and injected into a patient. With appropriate imaging techniques the tumor can be located using the radioactively conjugated antibody. If the amount of radioactivity attached to the antibody is increased considerably, or the antibody or antibody fragment is conjugated to a toxin, the conjugate can be used to kill tumor cells *in vivo.* The antibody or fragment provides the targeting function, and the toxin or radioactivity kills the cells which are targeted by the antibody.

### Example 9

### Gene Therapy

The gene corresponding to tumor specific proteins identified by the method of the present invention may be isolated and identified by techniques known to those skilled in the art. The gene can then be inactivated by molecular biological techniques and replaced into the body by gene therapy. Alternatively, anti-sense molecules can be made to genes of the tumor specific markers, and the anti-sense molecules can be used as therapeutics. By either of the above methods known to those skilled in the art, the tumor specific gene expression is decreased.

## Claims

1. A method of diagnosing or monitoring in vitro the progression of esophageal cancer disease in a human patient suffering from an esophageal tumor comprising an adenocarcinoma, the method comprising:
a) determining the amount of at least one human protein which is over-expressed in esophageal tumours comprising adenocarcinomas, compared to normal esophageal tissues, in a sample from the patient, the protein giving on 2-D gel electrophoresis of esophageal tumour tissue an N-terminal amino acid sequence
(1) Gly Ser Thr Arg Ile Pro Lys Ile Gln Lys Leu Leu Gln Asp and molecular weight in the gel of 37 kD (IPG 29, Fig. 20);
an N-terminal amino acid sequence:
(2) Gln Val Lys Gly Lys Lys Asn Ile Arg Ala Thr Glu and molecular weight in the gel of 25 kD
(IEF 42 = IPG 51, Fig. 20);
N-terminal amino acid sequences:
(3a) Gly Asn Trp Lys Met Asn Gln Arg and (3b) Val Gly Gly Asn Trp Lys Met Asn and molecular weight in the gel of 26 kD (IPG 53, Fig. 20);
or an N-terminal amino acid sequence:
(4) Xaa Val Phe Val Xaa Arg Xaa Met Lys,
where Xaa is an unknown or incompletely determined amino acid, the first Xaa being Ala, Ser or Gln, the second Xaa being Phe or Gly and the third unknown and a molecular weight in the gel of 19kD (IPG 56, Fig. 20); and
b) correlating the amount of the protein with the presence or stage of the disease.

2. A method according to Claim 1, wherein the determination comprises an immunological assay.

3. A method according to Claim 1, wherein the determination comprises 2-D gel electrophoresis.

4. A conjugated antibody or antibody fragment to a protein defined in Claim 1 with (a) a radioactive tag, for use in an *in vivo* imaging technique to locate a tumour within a human patient or (b) a toxin, for use in killing tumour cells within a human patient.

## Patentansprüche

1. Eine Methode zur *in vitro* Diagnostizierung oder Beobachtung des Fortschreitens einer Ösophaguskarzinom-Erkrankung bei einem menschlichen Patienten, der an einem Tumor im Ösophagus leidet, einschließlich eines Adenokarzinoms, wobei die Methode beinhaltet:
a) die Bestimmung der Menge von mindestens einem menschlichen Protein in einer Probe des Patienten, das in ösophagialen Tumoren, einschließlich Adenokarzinome, überexprimiert wird im Vergleich zu normalen ösophagialen Geweben, wobei das Protein bei einer 2-D Gelelektrophorese von Ösophagialtumor-Gewebe die N-terminale Aminosäuresequenz hat
(1) Gly Ser Thr Arg lie Pro Lys Ile Gln Lys Leu Leu Gln Asp und ein Molekulargewicht im Gel von 37 kD (IPG 29, Fig. 20);
eine N-terminale Aminosäuresequenz:
(2) Gln Val Lys Gly Lys Lys Asn Ile Arg Ala Thr Glu und ein Molekulargewicht im Gel von 25 kD
(IEF 42 = IPG 51, Fig. 20);
N-terminale Aminosäuresequenzen:
(3a) Gly Asn Trp Lys Met Asn Gln Arg und (3b) Val Gly Gly Asn Trp Lys Met Asn und ein Molekulargewicht im Gel von 26 kD (IPG 53, Fig. 20);
oder eine N-terminale Aminosäuresequenz:
(4) Xaa Val Phe Val Xaa Arg Xaa Met Lys,
wobei Xaa eine unbekannte oder eine unvollständig bestimmte Aminosäure ist, dabei kann das erste Xaa Ala, Ser oder Gly sein, das zweite Xaa Phe oder Gly und das dritte unbekannt, und ein Molekulargewicht im Gel von 19 kD (IPG 56, Fig. 20); und
b) die Korrelation zwischen der Protein-Menge und der Ausprägung oder dem Stadium der Erkrankung.

2. Eine Methode gemäß Anspruch 1, wobei die Bestimmung eine immunologische Untersuchung beinhaltet.

3. Eine Methode gemäß Anspruch 1, wobei die Bestimmung eine 2-D Gelelektrophorese beinhaltet .

4. Ein konjugierter Antikörper oder ein Antikörper-Fragment zu einem Protein, das in Anspruch 1 definiert wird, mit (a) einer radioaktiven Markierung zur Verwendung in einer *in vivo* Visualisierungstechnik um einen Tumor in einem menschlichen Patienten zu lokalisieren oder (b) einem Toxin, das zum Töten von Tumorzellen in einem menschlichen Patienten verwendet werden kann.

## Revendications

1. Procédé de diagnostic ou de surveillance in vitro de la progression d'un cancer de l'oesophage chez un patient humain souffrant d'une tumeur de l'oesophage comprenant un adénocarcinome, le procédé comprenant:
a) la détermination de la quantité d'au moins une protéine humaine qui est sur-exprimée dans des tumeurs de l'oesophage, comprenant des adénocarcinomes, par rapport à des tissus oesophagiens normaux, dans un échantillon prélevé sur le patient, la protéine donnant, par électrophorèse sur gel bidimensionnelle de tissus de tumeurs de l'oesophage, une séquence d'acides aminés N-terminale:
(1) Gly Ser Thr Arg Ile Pro Lys Ile Gln Lys Leu Leu Gln Asp et une masse moléculaire dans le gel de 37 kD (IPG 29, figure 20) ;
une séquence d'acides aminés N-terminale :
(2) Gln Val Lys Gly Lys Lys Asn Ile Arg Ala Thr Glu et une masse moléculaire dans le gel de 25 kD (FIE 42 = IPG 51, figure 20) ;
des séquences d'acides aminés N-terminales :
(3a) Gly Asn Trp Lys Met Asn Gln Arg et (3b) Val Gly Gly Asn Trp Lys Met Asn et une masse moléculaire dans le gel de 26 kD (IPG 53, figure 20) ;
ou une séquence d'acides aminés N-terminale :
(4) Xaa Val Phe Val Xaa Arg Xaa Met Lys,
où Xaa représente un acide aminé inconnu ou déterminé de manière incomplète, le premier Xaa étant Ala, Ser ou Gln, le deuxième Xaa étant Phe ou Gly et le troisième étant inconnu et une masse moléculaire dans le gel de 19 kD (IPG 56, figure 20) ; et
b) la corrélation de la quantité de la protéine avec la présence ou le stade de la maladie.

2. Procédé selon la revendication 1, dans lequel la détermination comprend un dosage immunologique.

3. Procédé selon la revendication 1, dans lequel la détermination comprend une électrophorèse sur gel bidimensionnelle.

4. Anticorps ou fragment d'anticorps conjugué à une protéine définie dans la revendication 1, avec (a) un marqueur radioactif pour une utilisation dans une technique d'imagerie *in vivo* pour localiser une tumeur chez un patient humain, ou (b) une toxine pour une utilisation dans la destruction de cellules tumorales chez un patient humain.
